# EUROPÄISCHE PATENTSCHRIFT

(11) **EP 1 695 146 B1**
(45) Veröffentlichungstag und Bekanntmachung des Hinweises auf die Patenterteilung: **16.04.2008**
(21) Anmeldenummer: 04803623.0
(22) Anmeldetag: 08.12.2004
(51) Int. Cl.: G03F 7/039, C07C 67/00

(54) **alpha-(1' -HYDROXYALKYL)ACRYLATE ENTHALTENDE BESCHICHTUNGSMASSEN**
COATING MATERIALS CONTAINING alpha-(1 -HYDROXYALKYL)ACRYLATES
MASSES DE REVETEMENT CONTENANT DES alpha-(1'-HYDROXYALKYL)ACRYLATES

(30) Priorität: 10.12.2003 DE 10358081
(43) Veröffentlichungstag der Anmeldung: 30.08.2006
(73) Patentinhaber: BASF SE, 67056 Ludwigshafen (DE)
(72) Erfinder: SCHWALM, Reinhold, 67157 Wachenheim (DE); BECK, Erich, 68526 Ladenburg (DE); HEISCHKEL, Yvonne, 68199 Mannheim (DE); GRUBER, Nick, 68161 Mannheim (DE); LARBIG, Harald, 67059 Ludwigshafen (DE)
(86) Internationale Anmeldenummer: PCT/EP2004/013948
(87) Internationale Veröffentlichungsnummer: WO 2005/057286

(56) Entgegenhaltungen:
- EP-A- 1 085 379
- US-A- 5 380 901
- PATENT ABSTRACTS OF JAPAN Bd. 2000, Nr. 14, 5. März 2001 (2001-03-05) & JP 2000 319228 A (NIPPON SHOKUBAI CO LTD), 21. November 2000 (2000-11-21)

## Beschreibung

Die vorliegende Erfindung betrifft Beschichtungsmassen, die α-(1'-Hydroxyalkyl)-acrylate enthalten, Verfahren zu deren Herstellung und deren Verwendung.

α-(1'-Hydroxyalkyl)acrylate sind herstellbar über die sog. Baylis-Hillman-Reaktion, in der Acrylate und Aldehyde miteinander umgesetzt werden.

US 5,380,901 beschreibt die Reaktion von Acrylaten mit para-Formaldehyd unter Bildung von etherverbrückten Diacrylaten sowie die Umsetzung von Diacrylaten mit Formaldehyd zu Di-(α-(1'-Hydroxyalkyl))acrylaten sowie die potentielle Verwendung solcher Monomere in beispielsweise Beschichtungen. Als Härtung wird lediglich Massepolymerisation beschrieben.

US-B1 6,482,568 beschreibt durch Excimer-Laser strahlungshärtbare Harzzusammensetzungen für Photoresiste, die aus speziellen Norbomen-Derivaten als Monomere aufgebaut sind und als weitere Monomere optional auch α-Hydroxymethylacrylate einpolymerisiert enthalten können.

Die dort beschriebenen speziellen Harzzusammensetzungen werden ebenfalls lediglich durcht Massepolymerisation hergestellt und die polymerisierten Harze sind nur die Härtung mit bestimmten Wellenlängen in der Lithographie konzipiert und nicht breit anwendbar.

Aufgabe der vorliegenden Erfindung war es, verbesserte Monomere für Dual-Cure-Beschichtungsmassen zur Verfügung zu stellen.

Die Aufgabe wurde gelöst durch Verbindungen der Formel (V), 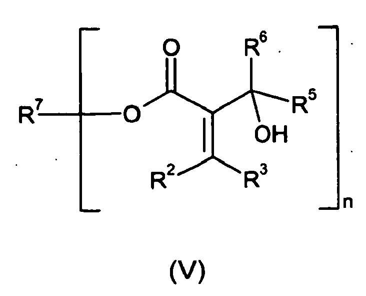 worin
R² und R³ unabhängig voneinander C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können,
R² und/oder R³ zusätzlich Wasserstoff, gegebenenfalls durch Aryl, Alkyl, Aryloxy, Alkyloxy, Heteroatome und/oder Heterocyclen substituiertes C₁ - C₁₈-Alkoxy oder -COOR⁴,
R² kann zusätzlich zusammen mit R¹ einen Ring bilden, in diesem Fall kann R² eine Carbonylgruppe bedeuten, so daß die Gruppe COOR¹ und R² gemeinsam eine Säureanhydridgruppe -(CO)-O-(CO)- bilden,
R⁴ C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können,
R⁵ und R⁶ unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder können gemeinsam einen Ring bilden,
n eine positive ganze Zahl von 3 bis 10,
R⁷ einen n-wertigen organischen Rest mit 1 bis 50 Kohlenstoffatomen, der unsubstituiert oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxy oder hydroxysubstituiertem C₁-C₈-Alkyl substituiert sein und/oder eine oder mehrere -(CO)-, -O(CO)O-, -(NH)(CO)O-, -O(CO)(NH)-, -O(CO)- oder -(CO)O-Gruppen aufweisen kann.

Mit dem Begriff "Dual Cure" beziehungsweise "Multi Cure" ist im Rahmen dieser Schrift ein Härtungsprozeß bezeichnet, der über zwei beziehungsweise mehr als zwei Mechanismen erfolgt und zwar beispielsweise ausgewählt aus strahlungs-, feuchtigkeits-, chemisch, oxidativ und/oder thermisch härtend, bevorzugt ausgewählt aus strahlungs-, feuchtigkeits-, chemisch und/oder thermisch härtend, besonders bevorzugt ausgewählt aus strahlungs-, chemisch und/oder thermisch härtend und ganz besonders bevorzugt strahlungs- und chemisch härtend.

Strahlungshärtung im Sinne dieser Schrift ist definiert als die Polymerisation von polymerisierbaren Verbindungen infolge einer elektromagnetischen und/oder korpuskularen Strahlung, bevorzugt UV-Licht im Wellenlängenbereich von λ=200 bis 700 nm und/oder Elektronenstrahlung im Bereich von 150 bis 300 keV und besonders bevorzugt mit einer Strahlungsdosis von mindestens 80, bevorzugt 80 bis 3000 mJ/cm².

Chemische Härtung im Sinne dieser Schrift ist definiert als die Polymerisation von polymerisierbaren Verbindungen infolge einer Reaktion von Hydroxygruppen (-OH) mit gegenüber Hydroxy (-OH) reaktiven Gruppen, beispielsweise Isocyanaten, verkappten Isocyanaten, Epoxiden, Carbonaten oder Aminoplasten, bevorzugt Isocyanaten, Epoxiden oder Aminoplasten, besonders bevorzugt Isocyanaten oder Epoxiden und ganz besonders bevorzugt Isocyanaten.

Geeignete α-(1'-Hydroxyalkyl)acrylate (A) können eine oder mehrere, beispielsweise 1 bis 10, bevorzugt 1 bis 6, besonders bevorzugt 1 bis 4, ganz besonders bevorzugt 2 bis 4 und insbesondere 3 bis 4 α-(1'-Hydroxyalkyl)acrylatgruppen tragen,

Das zahlenmittlere Molekulargewicht Mₙ dieser Verbindungen (A), bestimmt durch Gelpermeationschromatographie mit Tetrahydrofuran als Elutionsmittel und polystyrol als Standard, kann beispielsweise bis zu 10000, bevorzugt bis zu 5000, besonders bevorzugt zwischen 100 und 2000 und insbesondere zwischen 100 und 1000 g/mol betragen.

Beispiele für solche α-(1'-Hydroxyalkyl)acrylate (A) sind Verbindungen, die erhältlich sind durch Umsetzung eines ein- oder mehrfunktionellen Acrylats mit einer ein- oder mehrfunktionellen Carbonylverbindung.

Beispiele für Carbonylverbindungen sind Aldehyde oder Ketone, bevorzugt Aldehyde.

Dabei kann es sich um die Umsetzung eines monofunktionellen Acrylats (I) mit einer monofunktionellen Carbonylverbindung (II) handeln.

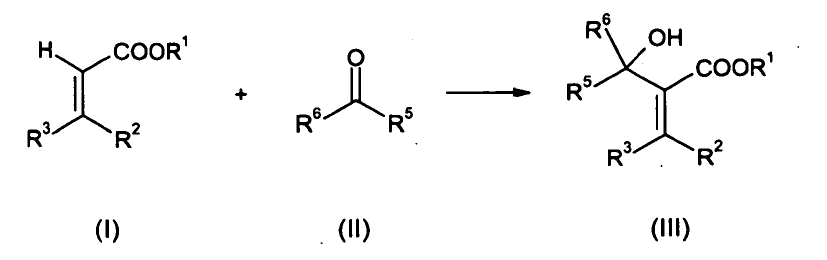

Weiterhin kann es sich um die Umsetzung eines mehrfunktionellen Acrylats (IV) mit einer monofunktionellen Carbonylverbindung (II) handeln.

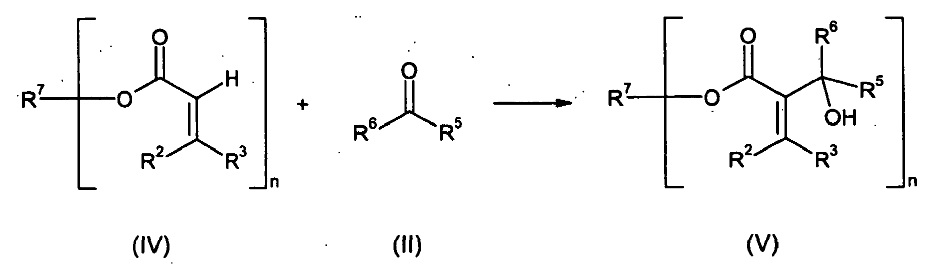

Weiterhin kann es sich um die Umsetzung eines mehrfunktionellen Acrylats (I) mit einer mehrfunktionellen Carbonylverbindung (VI) handeln.

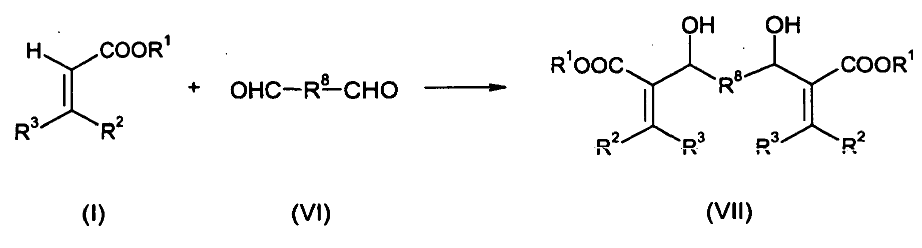

Darin bedeuten
R¹, R² und R³ unabhängig voneinander C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können,
R² und/oder R³ zusätzlich Wasserstoff, gegebenenfalls durch Aryl, Alkyl, Aryloxy, Alkyloxy, Heteroatome und/oder Heterocyclen substituiertes C₁ - C₁₈-Alkoxy oder -COOR⁴, R² kann zusätzlich zusammen mit R¹ einen Ring bilden, in diesem Fall kann R² eine Carbonylgruppe bedeuten, so daß die Gruppe COOR¹ und R² gemeinsam eine Säureanhydridgruppe -(CO)-O-(CO)- bilden,
R⁴ hat die gleiche Bedeutung wie für R¹ aufgeführt, kann jedoch von diesem verschieden sein,
R⁵ und R⁶ unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder können gemeinsam einen Ring bilden,
n eine positive ganze Zahl von 2 bis 10,
R⁷ einen n-wertigen organischen Rest mit 1 bis 50 Kohlenstoffatomen, der unsubstituiert oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxy oder hydroxysubstituiertem C₁-C₈-Alkyl substituiert sein und/oder eine oder mehrere -(CO)-, -O(CO)O-, -(NH)(CO)O-, -O(CO)(NH)-. -O(CO)- oder-(CO)O-Gruppen aufweisen kann und
R⁸ bedeutet unsubstituiertes oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₆-C₁₂-Arylen, C₃-C₁₂-Cycloalkylen, C₁-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, -O(CO)(NH)-, -O(CO)- oder -(CO)O-Gruppen unterbrochenes C₂-C₂₀-Alkylen oder eine Einfachbindung.

Darin bedeuten
gegebenenfalls durch Aryl, Alkyl, Aryloxy, Alkyloxy, Heteroatome und/oder Heterocyclen substituiertes C₁ - C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hetadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl,
2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl,
gegebenenfalls durch Aryl, Alkyl, Aryloxy, Alkyloxy, Heteroatome und/oder Heterocyclen substituiertes C₁ - C₁₈-Alkoxy beispielsweise Methoxy, Ethoxy, n-Propyloxy, iso-Propyloxy, n-Butyloxy, iso-Butyloxy, sek.-Butyloxy, tert.-Butyloxy, 6-Hydroxy-1,4-dioxohexyl, 9-Hydroxy-1,4,7-trioxononyl, 12-Hydroxy-1,4,7,10-tetraoxododecyl, 6-Methoxy-1,4-dioxohexyl, 9-Methoxy-1,4,7-trioxononyl, 12-Methoxy-1,4,7,10-tetraoxododecyl, 6-Ethoxy-1,4-dioxohexyl, 9-Ethoxy-1,4,7-trioxononyl, 12-Ethoxy-1,4,7,10-tetraoxododecyl, 8-Hydroxy-1,5-dioxooctyl, 12-Hydroxy-1,5,9-trioxooctyl, 16-Hydroxy-1,5,9,13-tetraoxohexadecyl, 8-Methoxy-1,5-dioxooctyl, 12-Methoxy-1,5,9-trioxooctyl, 16-Methoxy-1,5,9,13-tetraoxohexadecyl, 8-Ethoxy-1,5-dioxooctyl, 12-Ethoxy-1,5,9-trioxooctyl, 16-Ethoxy-1,5,9,13-tetraoxohexadecyl, 10-Hydroxy-1,6-dioxodecyl, 15-Hydroxy-1,6,11-trioxopentadecyl, 10-Methoxy-1,6-dioxodecyl, 15-Methoxy-1,6,11-trioxopentadecyl, 10-Ethoxy-1,6-dioxodecyl oder 15-Ethoxy-1,6,11-trioxopentadecyl,
gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-rionyl, 14-Hydroxy-5,10-oxatetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxapentadecyl, 9-Ethoxy-5-oxa-nonyl oder 14-Ethoxy-5,10-oxa-tetradecyl.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befinden sich zwischen zwei Heteroatomen in der Regel mindestens ein Kohlenstoffatom, bevorzugt mindestens zwei.

Substituierte und unsubstituierte Iminogruppen können beispielsweise Imino-, Methylimino-, *iso*-Propylimino, n-Butylimino oder *tert*-Butylimino sein.

Weiterhin bedeutet
gegebenenfalls durch Aryl, Alkyl, Aryloxy, Alkyloxy, Heteroatome und/oder Heterocyclen substituiertes C₂ - C₁₈-Alkenyl beispielsweise Vinyl, 1-Propenyl, Allyl, Methallyl, 1,1-Dimethylallyl, 2-Butenyl, 2-Hexenyl, Octenyl, Undecenyl, Dodecenyl, Octadecenyl, 2-Phenylvinyl, 2-Methoxyvinyl, 2-Ethoxyvinyl, 2-Methoxyallyl, 3-Methoxyallyl, 2-Ethoxyallyl, 3-Ethoxyallyl oder 1- oder 2-Chlorvinyl,
gegebenenfalls durch Aryl, Alkyl, Aryloxy, Alkyloxy, Heteroatome und/oder Heterocyclen substituiertes C₆ - C₁₂-Aryl beispielsweise Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso-*Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlomaphthyl, Ethoxynaphthyl, 2,6-Dimethylphenyl, 2,4,6-Trimethylphenyl, 2,6-Dimethoxyphenyl, 2,6-Dichlorphenyl,
4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch Aryl, Alkyl, Aryloxy, Alkyloxy, Heteroatome und/oder Heterocyclen substituiertes C₅ - C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,
ein fünf- bis sechsgliedriger, Sauerstoff-, Stickstoff- und/oder Schwefelatome aufweisender Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl und
C₁ bis C₄-Alkyl beispielsweise Methyl, Ethyl, Propyl, Isopropyl, n-Butyl, sec-Butyl oder tert.-Butyl.

Die Anzahl der Substituenten in den angegebenen Resten ist nicht beschränkt. In der Regel beträgt sie bei Resten mit ein bis drei Kohlenstoffatomen bis zu 3 Substituenten, bevorzugt bis zu 2 und besonders bevorzugt bis zu einem. Bei Resten mit vier bis sechs Kohlenstoffatomen beträgt sie in der Regel bis zu 4 Substituenten, bevorzugt bis zu 3 und besonders bevorzugt bis zu einem. Bei Resten mit mehr als sieben Kohlenstoffatomen beträgt sie in der Regel bis zu 6 Substituenten, bevorzugt bis zu 4 und besonders bevorzugt bis zu zwei.

R¹ ist bevorzugt durch Aryl, Alkyl, Aryloxy, Alkyloxy, Heteroatome und/oder Heterocyclen substituiertes C₁ - C₁₈-Alkyl oder C₅ - C₁₂-Cycloalkyl, beispielsweise Methyl, Ethyl, Propyl, Isopropyl, n-Butyl, sec-Butyl, tert.-Butyl, Pentyl, Hexyl, Heptyl, Octyl, 2-Etylhexyl, 2-Hydroxyethyl, 2-Hydroxypropyl, 3-Hydroxypropyl, 4-Hydroxybutyl, 6-Hydroxyhexyl, Cyclopentyl, Cyclohexyl, Cyclooctyl, Cyclododecyl, Methylcyclopentyl, Dimethylcyclopentyl, Methylcyclohexyl, Dimethylcyclohexyl, Norbornyl oder Norbornenyl, besonders bevorzugt ist R¹ Methyl, Ethyl, Propyl, Isopropyl, n-Butyl, sec-Butyl, tert.-Butyl, Pentyl, Hexyl, Heptyl, Octyl, 2-Etylhexyl, 2-Hydroxyethyl, 2-Hydroxypropyl, 3-Hydroxypropyl, 4-Hydroxybutyl oder 6-Hydroxyhexyl, ganz besonders bevorzugt Methyl, Ethyl, Propyl, Isopropyl, n-Butyl, sec-Butyl, tert.-Butyl oder 2-Etylhexyl und insbesondere Methyl, Ethyl, n-Butyl oder 2-Etylhexyl.

R² ist bevorzugt Wasserstoff, durch Aryl, Alkyl, Aryloxy, Alkyloxy, Heteroatome und/oder Heterocyclen substituiertes C₁ - C₁₈-Alkyl oder eine Carbonylgruppe, die mit R¹ verbunden ist, so daß die Gruppe COOR¹ und R² gemeinsam eine Säureanhydridgruppe -(CO)-O-(CO)- bilden, besonders bevorzugt Wasserstoff, Methyl, Ethyl, Propyl, Isopropyl, n-Butyl, sec-Butyl oder tert.-Butyl, ganz besonders bevorzugt Wasserstoff oder Methyl und insbesondere Wasserstoff.

R³ ist bevorzugt Wasserstoff, durch Aryl, Alkyl, Aryloxy, Alkyloxy, Heteroatome und/oder Heterocyclen substituiertes C₁ - C₁₈-Alkyl, besonders bevorzugt Wasserstoff, oder C₁-C₄-Alkyl, worunter im Rahmen dieser Schrift Methyl, Ethyl, Propyl, Isopropyl, n-Butyl, sec-Butyl oder tert.-Butyl verstanden wird, ganz besonders bevorzugt Wasserstoff oder Methyl und insbesondere Wasserstoff.

R⁴ ist bevorzugt Methyl, Ethyl, Propyl, Isopropyl, n-Butyl, sec-Butyl oder tert.-Butyl, besonders bevorzugt Methyl oder Ethyl.

R⁵ und R⁶ sind unabhängig voneinander bevorzugt Wasserstoff, durch Aryl, Alkyl, Aryloxy, Alkyloxy, Heteroatome und/oder Heterocyclen substituiertes C₁ - C₁₈-Alkyl, C₂ - C₁₈-Alkenyl, C₆ - C₁₂-Aryl oder C₅ - C₁₂-Cycloalkyl, besonders bevorzugt Wasserstoff, durch Aryl, Alkyl, Aryloxy, Alkyloxy, Heteroatome und/oder Heterocyclen substituiertes C₁ - C₁₈-Alkyl oder C₆ - C₁₂-Aryl, ganz besonders bevorzugt Wasserstoff, Methyl, Ethyl, Propyl, Isopropyl, n-Butyl, sec-Butyl, tert.-Butyl, Phenyl, Benzyl, Tolyl, o-, m- oder p-Xylyl, 2-, 3- oder 4-Methoxyphenyl, 2-, 3- oder 4-Chlorphenyl oder 2-, 3- oder 4-Nitrophenyl und insbesondere Wasserstoff, Methyl, Ethyl, Propyl, Isopropyl oder Phenyl.

Bevorzugt ist mindestens einer der beiden Reste R⁵ und R⁶ Wasserstoff.

R⁷ ist bevorzugt ein organischer Rest, abgeleitet von einem n-wertigen Alkohol durch Entfernung von n Hydroxygruppen, beispielsweise abgeleitet von zwei- bis zehnwertigen Alkoholen, besonders bevorzugt abgeleitet von zwei- bis sechswertigen Alkoholen, ganz besonders bevorzugt abgeleitet von zwei- bis vierwertigen Alkoholen und insbesondere abgeleitet von zwei- bis dreiwertigen Alkoholen.
R⁸ ist bevorzugt bedeutet unsubstituiertes oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₆-C₁₂-Arylen, C₃-C₁₂-Cycloalkylen oder C₁-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, -O(CO)(NH)-, -O(CO)- oder -(CO)O-Gruppen unterbrochenes C₂-C₂₀-Alkylen oder eine Einfachbindung, besonders bevorzugt unsubstituiertes oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₁-C₂₀-Alkylen oder eine Einfachbindung und ganz besonders bevorzugt unsubstituiertes oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₁-C₂₀-Alkylen.

Beispiele für Verbindungen (I) sind Acrylsäuremethylester, Acrylsäureethylester, Acrylsäure-n-propylester, Acrylsäure-iso-propylester, Acrylsäure-n-butylester, Acrylsäuresek-butylester, Acrylsäure-tert-butylester, Acrylsäure-2-ethylhexylester, 2-Hydroxyethylacrylat, 5-Hydroxy-3-oxa-pentylacrylat, 2-Hydroxypropylacrylat, 3-Hydroxypropylacrylat, 4-Hydroxybutylacrylat, 6-Hydroxyhexylacrylat, Dihydrodicyclopentadienylacrylat, Norbornylacrylat, Cyclohexylacrylat, Cyclopentylacrylat, Cyclododecylacrylat, Phenylacrylat, Crotonsäuremethylester, Crotonsäureethylester, Maleinsäureanhydrid, Maleinsäuredimethylester, Maleinsäurediethylester, Maleinsäuredi-n-butylester, Fumarsäuredimethylester oder Fumarsäurediethylester.

Bevorzugte Verbindungen (I) sind Acrylsäuremethylester, Acrylsäureethylester, Acrylsäure-n-propylester Acrylsäure-iso-propylester, Acrylsäure-n-butylester, Acrylsäuresek-butylester, Acrylsäure-tert-butylester und Acrylsäure-2-ethylhexylester.

Besonders bevorzugte Verbindungen (I) sind Acrylsäuremethylester, Acrylsäureethylester, Acrylsäure-n-butylester und Acrylsäure-2-ethylhexylester.

Beispiele für Verbindungen (II) sind Formaldehyd, Acetaldehyd, Propionaldehyd, n-Butyraldehyd, iso-Butyraldehyd, Heptanal, Nonanal, Cyclopentylaldehyd; Cyclohexylaldehyd, Benzaldehyd, 3-Chlorbenzaldehyd, 4-Chlorbenzaldehyd, 3-Hydroxybenzaldehyd, 4-Hydroxybenzaldehyd, 3-Methoxybenzaldehyd, 4-Methylbenzaldehyd, Phenylacetaldehyd, Salicylaldehyd, Chloralhydrat, 4-Dimethylaminobenzaldehyd, Furfural, 2-Nitrobenzaldehyd, Vanilin, Anisaldehyd, Zimtaldehyd, Pyridincarbaldehyd, Hydroxypivalinaldehyd, Dimethylolpropionaldehyd, Dimethylolbutyraldehyd, Trimethylolacetaldehyd, Aceton, Ethylmethylketon, Diethylketon, Methylvinylketon, iso-Butylmethylketon, Acetophenon, Propiophenon, Benzophenon, Cyclopentanon, Cyclohexanon oder Cyclododecanon.
Bevorzugte Verbindungen (II) sind die aufgelisteten Aldehyde, besonders bevorzugt sind Formaldehyd, Acetaldehyd, Propionaldehyd, n-Butyraldehyd, iso-Butyratdehyd, Benzaldehyd, 3-Hydroxybenzaldehyd, 4-Hydroxybenzaldehyd, Hydroxypivalinaldehyd, Dimethylolpropionaldehyd, Dimethylolbutyraldehyd und Trimethylolacetaldehyd, ganz besonders bevorzugt sind Formaldehyd, Acetaldehyd, Propionaldehyd, n-Butyraldehyd, iso-Butyraldehyd, 3-Hydroxybenzaldehyd, 4-Hydroxybenzaldehyd und Dimethylolbutyraldehyd und insbesondere Formaldehyd.

Es ist zur Herstellung nicht-etherverbrückter Baylis-Hillman-Produkte vorteilhaft Aldehyde in freier Form einzusetzen, d.h. die Bildung von Formalen dieser Aldehyde der Formel (R⁵-CHO)_{w}, worin w eine positive ganze Zahl ist, durch Einsatz geeigneter Aldehyde bzw. Wahl geeigneter Lösungsmittel zurückzudrängen. Während in US 5,380,901 aufgrund des Einsatzes von para-Formaldehyd bzw. para-Formaldehyd/DMSO, in dem die Bildung von Formalen nicht unterdrückt ist, durchweg etherverbrückte Systeme erhalten werden, ist es vorteilhaft Aldehyde mit einem hohen Anteil niedriger Formale einzusetzen, beispielsweise w ≤ 20, bevorzugt w ≤ 10 und besonders bevorzugt w ≤ 5.

Der Anteil dieser niedrigen Formale sollte bezogen auf die Gesamtmenge Aldehyd beispielsweise mindestens 50 % betragen, bevorzugt mindestens 60 %, besonders bevorzugt mindestens 70 % und ganz besonders bevorzugt mindestens 80 %.

Dies wird im Fall von Formaldehyd beispielsweise dadurch erreicht, daß man Formaldehyd in Form von wäßrigen Lösungen einsetzt, beispielsweise nicht mehr als 49 %ig und bevorzugt bis zu 37 %ig.

Durch diese Maßnahmen ist es möglich, den Anteil etherverbrückter Baylis-Hillman Produkte zurückzudrängen. Dieser Anteil wird bestimmt als molarer Anteil der Aldehyd-Äquivalente in Etherverbrückungen (-CHR⁵-O-CHR⁵-) an der Summe der Baylis-Hillman Produkte, also Etherverbrückungen und endständige -CHR⁵OH-Gruppen.

Die Etherverbrückungen entsprechen somit 2 Moläquivalenten Aldehyd R⁵-CHO, wohingegen die endständigen -CHR⁵OH-Gruppen einem Moläquivalent Aldehyd entsprechen.

Die Bestimmung der Anteile der Gruppen kann beispielsweise über NMR-Spektroskopie erfolgen. Im Fall von Formaldehyd erscheint in ¹H-NMR-Spektren in CDCl₃ die CH₂-O-CH₂-Gruppe als Singulett bzw. durch Allylkopplung aufgespaltenes Singulett bei ca. δ=4,22 ppm (s. US 5,380,901) und die CH₂OH-Gruppe bei ca. 4,30 ppm oder in ¹³C-NMR-Spektren in CDCl₃ die CH₂-O-CH₂-Gruppe bei ca. δ=68,7 ppm und die CH₂OH-Gruppe bei ca. 62,0 ppm.

Durch die oben beschriebenen erfindungsgemäßen Maßnahmen des Einsetzens von Aldehyden mit einem geringen Anteil an Formalen kann der Anteil der Etherverbrükkungen in der Regel auf 50% oder weniger, bevorzugt auf 40% oder weniger, besonders bevorzugt auf nicht mehr als 33%, ganz besonders bevorzugt auf nicht mehr als 25% und insbesondere auf nicht mehr als 15% gesenkt werden.

Dahingegen beträgt der Anteil an Etherverbrückungen in dem in US 5,380,901 Spalte 5 dargestellten Silikondiacrylat mit n=2 und 95% x=-CH₂OH (US 5,380,901, Spalte 5, Zeile 57-59) etwa 69%. Ein hoher Anteil endständiger OH-Gruppen ist jedoch in der Dual-Cure-Härtung für die Reaktion mit gegenüber OH reaktiven Gruppen vorteilhaft.

Beispiele für Verbindungen (IV) sind Ethylenglykoldiacrylat, 1,2-Propandioldiacrjrlat, 1,3-Propandioldiacrylat, 1,4-Butandioldiacrylat, 1,3-Butandioldiacrylat, 1,5-Pentandioldiacrylat, 1,6-Hexandioldiacrylat, 1,8-Octandioldiacrylat, Neopentylglykoldiacrylat, 1,1-, 1,2-, 1,3- und 1,4-Cyclohexandimethanoldiacrylat, 1,2-, 1,3-oder 1,4-Cyclohexandioldiacrylat, Trimethylolpropantriacrylat, Ditrimethylolpropanpenta- oder -hexaacrylat, Pentaerythrittri- oder -tetraacrylat, Glycerindi- oder -triacrylat, sowie Di- und Polyacrylate von Zuckeralkoholen, wie beispielsweise Sorbit, Mannit, Diglycerol, Threit, Erythrit, Adonit (Ribit), Arabit (Lyxit), Xylit, Dulcit (Galactit), Maltit oder Isomalt, oder von Polyesterpolyolen, Polyetherolen, Poly-THF mit einer Molmasse zwischen 162 und 2000, Poly-1,3-Propandiol mit einer Molmasse zwischen 134 und 1178, Polyethylenglykol mit einer Molmasse zwischen 106 und 898, sowie Urethanacrylate oder Polycarbonatacrylate.

Weitere Beispiele sind Acrylate von Verbindungen der Formel (IVa) bis (IVc), 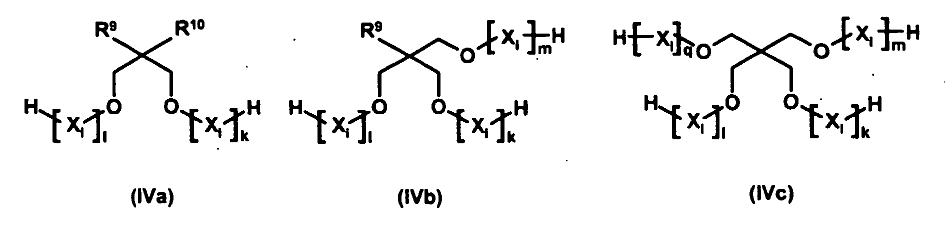 worin
R⁹ und R¹⁰ unabhängig voneinander Wasserstoff oder C₁- C₁₈-Alkyl,
k, l, m, q unabhängig voneinander je für eine ganze Zahl von 1 bis 10, bevorzugt 1 bis 5 und besonders bevorzugt 1 bis 3 steht und
jedes Xᵢ für i = 1 bis k, 1 bis l, 1 bis m und 1 bis q unabhängig voneinander ausgewählt sein kann aus der Gruppe -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- und -CHPh-CH₂-O-, bevorzugt aus der Gruppe -CH₂-CH₂-O-, -CH₂-CH(CH₃)-O- und -CH(CH₃)-CH₂-O-, und besonders bevorzugt -CH₂-CH₂-O-,
worin Ph für Phenyl und Vin für Vinyl steht.

Bevorzugt handelt es sich dabei um Acrylate von ein- bis zwanzigfach und besonders bevorzugt drei- bis zehnfach ethoxyliertem, propoxyliertem oder gemischt ethoxyliertem und propoxyliertem und insbesondere ausschließlich ethoxyliertem Neopentylglykol, Trimethylolpropan, Trimethylolethan oder Pentaerythrit.

Bevorzugte Verbindungen (IV) sind Ethylenglykoldiacrylat, 1,2-Propandioldiacrylat, 1,3-Propandioldiacrylat, 1,4-Butandioldiacrylat, 1,6-Hexandioldiacrylat, Trimethylolpropantriacrylat, Pentaerythrittetraacrylat, Polyestepolyolenacrylate, Polyetherolacrylate und Triacrylat von ein- bis zwanzigfach ethoxyliertem Trimethylolpropan.

Besonders bevorzugte Verbindungen sind 1,4-Butandioldiacrylat, 1,6-Hexandioldiacrylat,-Trimethylolpropantriacrylat, Pentaerythrittetraacrylat und Triacrylat von ein- bis zwanzigfach ethoxyliertem Trimethylolpropan.

Polyesterpolyole, sind z.B. aus Ullmanns Encyklopädie der technischen Chemie, 4. Auflage, Band 19, S. 62 bis 65 bekannt. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, araliphatisch, aromatisch oder heterocyclisch sein und gegebenenfalls, z.B. durch Halogenatome, substituiert und/oder ungesättigt sein. Als Beispiele hierfür seien genannt:
Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, Korksäure, Azelainsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäureanhydrid, dimere Fettsäuren, deren Isomere und Hydrierungsprodukte sowie veresterbare Derivate, wie Anhydride oder Dialkylester, beispielsweise C₁-C₄-Alkylester, bevorzugt Methyl-, Ethyl- oder n-Butylester, der genannten Säuren eingesetzt werden. Bevorzugt sind Dicarbonsäuren der allgemeinen Formel HOOC-(CH₂)_{y}-COOH, wobei y eine Zahl von 1 bis 20, bevorzugt eine gerade Zahl von 2 bis 20 ist, besonders bevorzugt Bernsteinsäure, Adipinsäure, Sebacinsäure und Dodecandicarbonsäure.
   Als mehrwertige Alkohole kommen zur Herstellung der Polyesterole in Betracht 1,2-Propandiöl, Ethylenglykol, 2,2-Dimethyl-1,2-Ethandiol, 1,3-Propandiol, 1,2-Butandiol, 1,3-Butandiol, 1,4-Butandiol, 3-Methylpentan-1,5-diol, 2-Ethylhexan-1,3-diol, 2,4-Diethyloctan-1,3-diol, 1,6-Hexandiol, Poly-THF mit einer Molmasse zwischen 162 und 2000, Poly-1,3-propandiol mit einer Molmasse zwischen 134 und 1178, Poly-1,2-propandiol mit einer Molmasse zwischen 134 und 898, Polyethylenglykol mit einer Molmasse zwischen 106 und 458, Neopentylglykol, Hydroxypivalinsäureneopentylglykolester, 2-Ethyl-1,3-Propandiol, 2-Methyl-1,3-Propandiol, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, Trimethylolbutan, Trimethylolpropan, Trimethylolethan, Neopentylglykol, Pentaerythrit, Glycerin, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit oder Isomalt.

Bevorzugt sind Alkohole der allgemeinen Formel HO-(CH₂)ₓ-OH, wobei x eine Zahl von 1 bis 20, bevorzugt eine gerade Zahl von 2 bis 20 ist. Bevorzugt sind Ethylenglycol, Butan-1,4-diol, Hexan-1,6-diol, Octan-1,8-diol und Dodecan-1,12- diol. Weiterhin bevorzugt ist Neopentylglykol.

Ferner kommen auch Polycarbonat-Diole, wie sie z.B. durch Umsetzung von Phosgen mit einem Überschuß von den als Aufbaukomponenten für die Polyesterpolyole genannten niedermolekularen Alkohole erhalten werden können, in Betracht.

Geeignet sind auch Polyesterdiole auf Lacton-Basis, wobei es sich um Homo- oder Mischpolymerisate von Lactonen, bevorzugt um endständige Hydroxylgruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, gamma-Butyrolacton und/oder Methyl-ε-caprolacton, 4-Hydroxybenzoesäure, 6-Hydroxy-2-naphthalinsäure oder Pivalolacton sowie deren Gemische. Geeignete Starterkomponenten sind z.B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden.

Beispiele für Verbindungen (VI) sind Glyoxal, Succinaldehyd, Glutaraldehyd, Capronaldehyd, Phthaldialdehyd oder Terephthaldialdehyd, bevorzugt Glyoxal.

Ein weiterer Gegenstand der vorliegenden Erfindung sind Verbindungen der Formel (V) in denen n mindestens 3 und bevorzugt 3 oder 4 ist. Ganz besonders bevorzugt sind solche Verbindungen, in denen der Rest R⁷ abgeleitet ist von gegebenenfalls alkoxyliertem Trimethylolpropan oder Pentaerythrit. Diese Verbindungen weisen durch das gleichzeitige Vorliegen von Acrylat und Hydroxy-Gruppen eine besonders gute Eignung für die Dual-Cure-Härtung auf. Es ist überraschend in Kenntnis der Lehre aus der US 5,380,901, daß trotz der höheren Dichte der Hydroxygruppen und deren räumlicher Nähe kein erhöhter Anteil an Etherverbrückungen wie oben definiert gefunden wurden.

Die Durchführung der Baylis-Hillman-Reaktion ist dem Fachmann an sich bekannt und beispielsweise beschrieben in H.M.R. Hoffmann, J. Rabe, Angew. Chem., Int. Ed. Engl., 22, 1983, 795 - 796 und 796 - 797, A. Foucaud, E. le Rouille, Synthesis, 1990, 787 - 789, Y. Fort, M.-C. Berthe, P. Caubere, Synthetic Communictions, 1992, 22(9), 1265 -1275 oder D. Basaviah, P. D. Rao und R. S. Hyma. Tetrahedron, 1996, 52(24), 8001- 8062.

Die Reaktion kann bei einer Temperatur zwischen 0 °C und 100 °C, bevorzugt 20 bis 80 °C und besonders bevorzugt 25 °C bis 60 °C durchgeführt werden.

Um Ketone zur Reaktion zu bringen kann es erforderlich sein, hohen Druck anzulegen, wie es beschrieben ist in D. Basaviah et al, a.a.O., S. 8004.

Als Katalysator für die Reaktion wird zumeist ein tertiäres Amin oder Phosphin, verwendet, beispielsweise Trimethylamin, Triethylamin, Tri-n-butylamin, Ethyl-di-*iso*-Propylamin, Methyl-di-*iso*-Propylamin, N-Methylmorpholin, N-Methylpiperidin, Triethanolamin, N,N-Dimethylethanolamin, 4-Dimethylaminopyridin, 1,5-Diazabicyclo[4.3.0]-non-5-en (DBN), 1,8-Diazabicyclo[5.4.0]-undec-7-en (DBU), Pyrrocolin, Chinuclidin, Chinidin, Trimethylphosphin, Triethylphosphin, Tri-n-Butylphosphin, Dimethylphenylphosphin, die in D. Basaviah et al, a.a.O., S. 8053- 8054 aufgeführten tertiären Amine oder Phosphine oder bevorzugt 1,4-Diaza-bicyclo[2,2,2]octan (DABCO) verwendet. Der Katalysator wird in der Regel in Mengen von 1 bis 50 mol% bzgl. Acrylgruppen eingesetzt, bevorzugt 5 - 50, besonders bevorzugt 10 - 40 und ganz besonders bevorzugt 15 - 30 mol%.

Die Stöchiometrie zwischen Acrylatgruppen und Carbonylverbindungen beträgt in der Regel 1 : 0,05 -1,5, bevorzugt 1 : 0,1 -1,3, besonders bevorzugt 1 : 0,2 -1,0 und ganz besonders bevorzugt 1 : 0,4 - 1,0.

Als Lösungsmittel können bevorzugt Wasser, Petrolether, Ligroin, Toluol, Benzol, Xylol, Tetrahydrofuran (THF), Diethylether, Dioxan, oder aber auch das verwendete Acrylat eingesetzt werden. Die Reaktion kann auch in Abwesenheit eines Lösungsmittels durchgeführt werden.
Wird das Acrylat als Lösungsmittel verwendet, so kann das entstehende Reaktionsgemisch, das sowohl das verwendete Acrylat als auch α-(1'-Hydroxyalkyl)acrylat enthält, aufgereinigt oder ohne Abtrennung des Acrylats als solches eingesetzt werden, wobei das Acrylat dann als Reaktivverdünner oder multifunktionelles Acrylat fungiert.

Auf eine Aufreinigung des Reaktionsgemisches kann verzichtet werden, selbstverständlich kann das Gemisch natürlich auch durch Destillation, Strippen, saure, alkalische oder neutrale Wäsche, Filtration oder dergleichen gereinigt werden.

In einer bevorzugten Ausführungsform wird die Carbonylverbindung im Verhältnis zu den Acrylatgruppen unterstöchiometrisch eingesetzt, so daß Reaktionsgemische erhalten werden, die das Baylis-Hillman-Produkt im Gemisch mit dem eingesetzten Acrylat enthalten. Derartige Gemische können mit Vorteil in Beschichtungsmassen für die Strahlungshärtung und/oder Dual-Cure-Härtung eingesetzt werden.

Ein weiterer Gegenstand der vorliegenden Erfindung sind Beschichtungsmassen, enthaltend
- mindestens eine Verbindung der Formel (V) oder der Formel (VII), und
- mindestens einen Photoinitiator (P).

Als Photoinitiatoren (P) können dem Fachmann bekannte Photoinitiatoren verwendet werden, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

Erfindungsgemäß werden darunter solche Photoinitiatoren verstanden, die unter Lichteinwirkung Radikale freisetzen und eine radikalische Reaktion, beispielsweise eine radikalische Polymerisation, starten können.

In Betracht kommen beispielsweise Phosphinoxide, Benzophenone, α-Hydroxy-alkyl-aryl-ketone, Thioxanthone, Anthrachinone, Acetophenone, Benzoine und Benzoinether, Ketale, Imidazole oder Phenylglyoxylsäuren und Gemische davon.

Phosphinoxide sind beispielsweise Mono- oder Bisacylphosphinoxide, wie z.B. Irgacure® 819 (Bis(2,4,6-Trimethylbenzoyl)phenylphosphinoxid), wie sie z.B. in EP-A 7 508, EP-A 57 474, DE-A 196 18 720, EP-A 495 751 oder EP-A 615 980 beschrieben sind, beispielsweise 2,4,6-Trimethylbenzoyldiphenylphosphinoxid (Lucirin^{®} TPO), Ethyl-2,4,6-trimethylbenzoylphenylphosphinat oder Bis(2,6-dimethoxybenzoyl)-2,4,4-trimethylpentylphosphinoxid,
Benzophenone sind beispielsweise Benzophenon, 4-Aminobenzophenon, 4,4'-Bis(dimethylamino)benzophenon, 4-Phenylbenzophenon, 4-Chlorbenzophenon, Michlers Keton, o-Methoxybenzophenon, 2,4,6-Trimethylbenzophenon, 4-Methylbenzophenon, 2,4-Dimethylbenzophenon, 4-lsopropylbenzophenon, 2-Chlorbenzophenon, 2,2'-Dichlorbenzophenon, 4-Methoxybenzophenon, 4-Propoxybenzophenon oder 4-Butoxybenzophenon,
α-Hydroxy-alkyl-aryl-ketone sind beispielsweise 1-Benzoylcyclohexan-1-ol (1-Hydroxy-cyclohexyl-phenylketon), 2-Hydroxy-2,2-dimethylacetophenon (2-Hydroxy-2-methyl-1-phenyl-propan-1 -on), 1-Hydroxyacetophenon, 1-[4-(2-Hydroxyethoxy)-phenyl]-2-hydroxy-2-methyl-1-propan-1-on oder Polymeres, das 2-Hydroxy-2-methyl-1-(4-isopropen-2-yl-phenyl)-propan-1-on einpolymerisiert enthält (Esacure® KIP 150)
Xanthone und Thioxanthone sind beispielsweise 10-Thioxanthenon, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon oder Chloroxanthenon,
Anthrachinone sind beispielsweise β-Methylanthrachinon, *tert*-Butylanthrachinon, Anthrachinoncarbonylsäureester, Benz[de]anthracen-7-on, Benz[a]anthracen-7,12-dion, 2-Methylanthrachinon, 2-Ethylanthrachinon, 2-*tert*-Butylanthrachinon, 1-Chloranthrachinon oder 2-Amylanthrachinon,
Acetophenone sind beispielsweise Acetophenon, Acetonaphthochinon, Valerophenon, Hexanophenon, α-Phenylbutyrophenon, p-Morpholinopropiophenon, Dibenzosuberon, 4-Morpholinobenzophenon, p-Diacetylbenzol, 4'-Methoxyacetophenon, α-Tetralon, 9-Acetylphenanthren, 2-Acetylphenanthren, 3-Acetylphenanthren, 3-Acetylindol, 9-Fluorenon, 1-Indanon, 1,3,4-Triacetylbenzol, 1-Acetonaphthon, 2-Acetonaphthon, 2,2-Dimethoxy-2-phenylacetophenon, 2,2-Diethoxy-2-phenylacetophenon, 1,1-Dichloracetophenon, 1-Hydroxyacetophenon, 2,2-Diethoxyacetophenon, 2-Methyl-1-[4-(methylthio)-phenyl]-2-morpholinopropan-1-on, 2,2-Dimethoxy-1,2-diphenylethan-2-on oder 2-Benzyl-2-dimethylamino-1-(4-morpholinophenyl)-butan-1-on,
Benzoine und Benzoinether sind beispielsweise 4-Morpholinodeoxybenzoin, Benzoin, Benzoin-iso-butylether, Benzoin-tetrahydropyranylether, Benzoin-methylether, Benzoin-ethylether, Benzoin-butylether, Benzoin-iso-propylether oder 7-H-Benzoin-methylether oder
Ketale sind beispielsweise Acetophenondimethylketal, 2,2-Diethoxyacetophenon, oder Benzilketale, wie Benzildimethylketal.

Phenylglyoxylsäuren sind beispielsweise in DE-A 198 26 712, DE-A 199 13 353 oder WO 98/33761 beschrieben.

Weiterhin verwendbare Photoinitiatoren sind beispielsweise Benzaldehyd, Methylethylketon, 1-Naphthaldehyd, Triphenylphosphin, Tri-o-Tolylphosphin oder 2,3-Butandion.

Typische Gemische umfassen beispielsweise 2-Hydroxy-2-Methyl-1-phenyl-propan-2-on und 1-Hydröxy-cyclohexyl-phenylketon, Bis(2,6-dimethoxybenzoyl)-2,4,4-trimethylpentylphosphinoxid und 2-Hydroxy-2-methyl-1-phenyl-propan-1-on, Benzophenon und 1-Hydroxy-cyclohexyl-phenylketon, Bis(2,6-dimethoxybenzoyl)-2,4,4-trimethylpentylphosphinoxid und 1-Hydroxy-cyclohexyl-phenylketon, 2,4,6-Trimethylbenzoyldiphenylphosphinoxid und 2-Hydroxy-2-methyl-1-phenyl-propan-1-on, 2,4,6-Trimethylbenzophenon und 4-Methylbenzophenon oder 2,4,6-Trimethylbenzophenon und 4-Methylbenzophenon und 2,4,6-Trimethylbenzoyldiphenylphosphinoxid.

Derartige erfindungsgemäße Beschichtungsmassen können erfindungsgemäß in der Strahlungshärtung angewendet werden.

Die erfindungsgemäßen Beschichtungsmassen können weiterhin mindestens einen Reaktivverdünner und/oder mindestens eine multifunktionelle, polymerisationsfähige Verbindung und/oder weitere lacktypische Additive enthalten.

Reaktivverdünner sind beispielsweise Ester der (Meth)acrylsäure mit Alkoholen, die 1 bis 20 C-Atome aufweisen, z.B. (Meth)acrylsäuremethylester, (Meth)acrylsäureethylester, (Meth)acrylsäurebutylester, (Meth)acrylsäure-2-ethylhexylester, 2-Hydroxyethylacrylat, 4-Hydroxybutylacrylat , Dihydrodicyclopentadienylacrylat, Vinylaromatische Verbindungen, z.B. Styrol, Divinylbenzol, α,β-ungesättigte Nitrile, z.B. Acrylnitril, Methacrylnitril, α,β-ungesättigte Aldehyde, z.B. Acrolein, Methacrolein, Vinylester, z.B. Vinylacetat, Vinylpropionat, halogenierte ethylenisch ungesättigte Verbindungen, z.B. Vinylchlorid, Vinylidenchlorid, konjugierte ungesättigte Verbindungen, z.B. Butadien, Isopren, Chloropren, einfach ungesättigte Verbindungen, z.B. Ethylen, Propylen, 1-Buten, 2-Buten, iso-Buten, cyclische einfach ungesättigte Verbindungen. z.B. Cyclopenten, Cyclohexen, Cyclododecen, N-Vinylformamid, Allylessigsäure, Vinylessigsäure, monoethylenisch ungesättigten Carbonsäuren mit 3 bis 8 C-Atomen sowie deren wasserlöslichen Alkalimetall-, Erdalkalimetall- oder Ammoniumsalze wie beispielsweise: Acrylsäure, Methacrylsäure, Dimethylacrylsäure, Ethacrylsäure, Maleinsäure, Citraconsäure, Methylenmalonsäure, Crotonsäure, Fumarsäure, Mesaconsäure und Itaconsäure, Maleinsäure, N-Vinylpyrrolidon, N-Vinyllactame, wie z.B. N-Vinylcaprolactam, N-Vinyl-N-Alkyl-carbonsäureamide oder N-Vinyl-carbonsäureamide, wie z. B. N-Vinylacetamid, N-Vinyl-N-methylformamid und N-Vinyl-N-methylacetamid oder Vinylether, z.B. Methylvinylether, Ethylvinylether, *n*-Propylvinylether, *iso*-Propylvinylether, n-Butylvinylether, *sek*-Butylvinylether, *iso*-Butylvinylether, *tert*-Butylvinylether, 4-Hydroxybutylvinylether, sowie Gemische davon.

(Meth)Acrylsäure steht in dieser Schrift für Methacrylsäure und Acrylsäure, bevorzugt für Acrylsäure.

Multifunktionelle, polymerisationsfähige Verbindungen sind bevorzugt multifunktionelle (Meth)acrylate, die mindestens 2, bevorzugt 3 - 10, besonders bevorzugt 3-6, ganz besonders bevorzugt 3-4 und insbesondere 3 (Meth)acrylatgruppen, bevorzugt Acrylatgruppen tragen.
Dies können beispielsweise Ester der (Meth)acrylsäure mit entsprechend mindestens zweiwertigen Polyalkoholen sein.

Derartige Polyalkohole sind beispielsweise mindestens zweiwertige Polyole, Polyether- oder Polyesterole oder Polyacrylatpolyole mit einer mittleren OH-Funktionalität von mindestens 2, bevorzugt 3 bis 10, geeignet.

Mindestens zweiwertige Polyalkohole sind beispielsweise solche, wie sie oben bei der Herstellung der Polyesterole aufgeführt sind.

Weitere geeignete mindestens zweiwertige Polyalkohole sind alkoxylierte mindestens zweiwertige Polyalkohole der oben aufgeführten Formeln (IVa), (IVb) oder (IVc).

Geeignete Alkylenoxide sind beispielsweise Ethylenoxid, Propylenoxid, iso-Butylenoxid, Vinyloxiran und/oder Styroloxid.

Die Alkylenoxidkette kann bevorzugt aus Ethylenoxid-, Propylenoxid- und/oder Butylenoxideinheiten zusammengesetzt sein. Eine solche Kette kann sich aus einer Spezies eines Alkylenoxides oder aus einem Gemisch von Alkylenoxiden zusammensetzen. Wird ein Gemisch verwendet, können die unterschiedlichen Alkylenoxideinheiten statistisch oder als Block oder Blöcke einzelner Spezies vorliegen. Bevorzugt ist als Alkylenoxid Ethylenoxid, Propylenoxid oder ein Gemisch daraus, besonders bevorzugt ist es Ethylenoxid oder Propylenoxid und ganz besonders bevorzugt Ethylenoxid.

Die Anzahl der Alkylenoxideinheiten in der Kette beträgt beispielsweise 1 bis 20, bevorzugt 1 bis 10, besonders bevorzugt 1 - 5 und insbesondere 1 - 3 und außergewöhnlich bevorzugt 1, bezogen auf die jeweiligen Hydroxygruppen des Polyalkohols.

Als Polyesterole kommen z.B. solche in Betracht, wie sie oben bereits aufgeführt sind.

Die Molekulargewichte Mₙ der Polyesterole bzw. Polyetherole liegen bevorzugt zwischen 100 und 4000 (Mₙ bestimmt durch Gelpermeationschromatographie mit Polystyrol als Standard und Tetrahydrofuran als Elutionsmittel).

Weitere multifunktionelle (Meth)acrylate können Polyester(meth)acrylate, Epoxy(meth)-acrylate, Urethan(meth)acrylate oder (meth)acrylierte Polyacrylate sein, wie sie oben als Acrylate von (IVa), (IVb) oder (IVc) aufgeführt sind. Anstelle der (Meth)acrylatgruppen können auch andere radikalisch oder kationisch polymerisierbare Gruppen eingesetzt werden.

Urethan(meth)acrylate sind z.B. erhältlich durch Umsetzung von Polyisocyanaten mit. Hydroxyalkyl(meth)acrylaten oder -vinylethern und gegebenenfalls Kettenverlängerungsmitteln wie Diolen, Polyolen, Diaminen, Polyaminen oder Dithiolen oder Polythiolen.

Bevorzugte multifunktionelle (Meth)acrylate sind Trimethylolpropantri(meth)acrylat, (Meth)Acrylate von ethoxyliertem und/oder propoxyliertem Trimethylolpropan, Pentaerythrit, Glycerin oder Di-Trimethylolpropan. Besonders bevorzugt sind Acrylate von ethoxyliertem und/oder propoxyliertem Trimethylolpropan oder Pentaerythrit.

Als weitere lacktypische Additive können beispielsweise Antioxidantien, Stabilisatoren, Aktivatoren (Beschleuniger), Füllmittel, Pigmente, Farbstoffe, antistatische Agentien, Flammschutzmittel, Verdicker, thixotrope Agentien, oberflächenaktive Agentien, Viskositätsmodifikatoren, Plastifizierer oder Chelatbildner verwendet werden.

Als Beschleuniger für die thermische Nachhärtung kann z.B. Zinnoctoat, Zinkoctoat, Dibutylzinnlaureat oder Diaza[2.2.2]bicyclooctan verwendet werden.

Weiterhin können ein oder mehrere photochemisch und/oder thermisch aktivierbare Initiatoren zugesetzt werden, z.B. Kaliumperoxodisulfat, Dibenzoylperoxid, Cyclohexanonperoxid, Di-tert.-Butylperoxid, Azobis-*iso*-butyronitril, Cyclohexylsulfonylacetylperoxid, Di-*iso*-propylpercarbonat, *tert*-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butylperoxid, Cumolhydroperoxid, Dicumylperoxid, t-Butylperbenzoat, silylierte Pinakole, die z. B. unter dem Handelsnamen AD-DID 600 der Firma Wacker kommerziell erhältlich sind oder Hydroxylgruppen-haltige Amin-N-Oxide, wie 2,2,6,6-Tetramethylpiperidin-N-oxyl, 4-Hydroxy-2,2,6,6-Tetramethylpiperidin-N-oxyl etc.

Weitere Beispiele geeigneter Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonit in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil^{®} der Fa. Degussa, Kieselerde, Talkum, Aluminiumsilikate, Magnesiumsilikate, Calciumcarbonate etc.
Geeignete Stabilisatoren umfassen typische UV-Absorber wie Oxanilide, Triazine und Benzotriazol (letztere erhältlich als Tinuvin^{®} -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Die erfindungsgemäßen Beschichtungsmassen sind mit-Vorteil für Dual- oder Multi-Cure Anwendungen verwendbar, wenn sie zusätzlich mindestens eine Verbindung (B) mit mindestens einer gegenüber Hydroxy (-OH) reaktiven Gruppe enthalten.

Verbindungen (B) mit mindestens einer gegenüber Hydroxy (-OH) reaktiven Gruppe können beispielsweise Isocyanate, verkappte Isocyanate, Epoxide, Carbonate oder Aminoplaste sein.

Isocyanate sind beispielsweise aliphatische, aromatische und cycloaliphatische Di- und Polyisocyanate mit einer NCO Funktionalität von mindestens 1,8, bevorzugt 1,8 bis 5 und besonders bevorzugt 2 bis 4 in Frage, sowie deren Isocyanurate, Biurete, Urethane, Allophanate und Uretdione.

Bei den Diisocyanaten handelt es sich bevorzugt um Isocyanate mit 4 bis 20 C-Atomen. Beispiele für übliche Diisocyanate sind aliphatische Diisocyanate wie Tetramethylendüsocyanat, Hexamethylendiisocyanat(1,6-Diisocyanatohexan), Octamethytendiisocyanat, Decamethylendüsocyanat, Dodecamethytendiisocyanat. Tetradecamethytendiisocyanat, Derivate des Lysindiisocyanates, Trimethylhexandüsocyanat oder Tetramethylhexandiisocyanat, cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isophorondüsocyanat), 1,3- oder 1,4-Bis(isocyanatomethyl)cyclohexan oder 2,4-, oder 2,6-Diisocyanato-1-methylcyclohexan sowie aromatische Diisocyanate wie 2,4- oder 2,6-Toluylendüsocyanat und deren Isomerengemische, m- oder p-Xylylendüsocyanat, 2,4'- oder 4,4'-DÜsocyanatodiphenylmethan und deren Isomerengemische, 1,3- oder 1,4-Phenylendiisocyanat, 1-Chlor-2,4-phenytendiisocyanat, 1,5-Naphthylendiisocyanat, Diphenylen-4,4'-diisocyanat, 4,4'-Diisocyanato-3,3'-dimethyldiphenyl, 3-Methyldiphenylmethan-4,4'-diisocyanat, Tetramethylxylylendiisocyanat, 1,4-Diisocyanatobenzol oder Diphenylether-4,4'-diisocyanat.

Es können auch Gemische der genannten Diisocyanate vorliegen.

Als Polyisocyanate kommen Isocyanuratgruppen aufweisende Polyisocyanate, Uretdiondiisocyanate, Biuretgruppen aufweisende Polyisocyanate, Urethan- oder Allophanatgruppen aufweisende Polyisocyanate, Oxadiazintriongruppen oder Iminooxadiazindiongruppen enthaltende Polyisocyanate, Uretonimin-modifizierte Polyisocyanate von geradlinigen oder verzweigten C₄-C₂₀-Alkylendiisocyanaten, cycloaliphatischen Diisocyanaten mit insgesamt 6 bis 20 C-Atomen oder aromatischen Diisocyanaten mit insgesamt 8 bis 20 C-Atomen oder deren Gemische in Betracht.

Die einsetzbaren Di- und Polyisocyanate haben bevorzugt einen Gehalt an isocyanatgruppen (berechnet als NCO, Molekulargewicht = 42) von 10 bis 60 Gew% bezogen auf das Di- und Polyisocyanat(gemisch), bevorzugt 15 bis 60 Gew% und besonders bevorzugt 20 bis 55 Gew%.

Bevorzugt sind aliphatische bzw. cycloaliphatische Di- und Polyisocyanate, z.B. die vorstehend genannten aliphatischen bzw. cycloaliphatischen Diisocyanate, oder deren Mischungen.

Besonders bevorzugt sind Hexamethylendiisocyanat, 1,3-Bis(isocyanatomethyl)-cyclohexan, Isophorondiisocyanat und Di(isocyanatocyclohexyl)methan, ganz besonders bevorzugt sind Isophorondiisocyanat und Hexamethylendiisocyanat, insbesondere bevorzugt ist Hexamethylendiisocyanat.

Weiterhin bevorzugt sind
1) Isocyanuratgruppen aufweisende Polyisocyanate von aromatischen, aliphatischen und/oder cycloaliphatischen Diisocyanaten. Besonders bevorzugt sind hierbei die entsprechenden aliphatischen und/oder cycloaliphatischen Isocyanato-Isocyanurate und insbesondere die auf Basis von Hexamethylendiisocyanat und Isophorondiisocyanat. Bei den dabei vorliegenden Isocyanuraten handelt es sich insbesondere um Tris-isocyanatoalkyl- bzw. Tris-isocyanatocycloalkyl-Isocyanurate, welche cyclische Trimere der Diisocyanate darstellen, oder um Gemische mit ihren höheren, mehr als einen Isocyanuratring aufweisenden Homologen. Die Isocyanato-Isocyanurate haben im allgemeinen einen NCO-Gehalt von 10 bis 30 Gew.%, insbesondere 15 bis 25 Gew.-% und eine mittlere NCO-Funktionalität von 2,6 bis 4,5.
2) Uretdiondiisocyanate mit aromatisch, aliphatisch und/oder cycloaliphatisch gebundenen Isocyanatgruppen, vorzugsweise aliphatisch und/oder cycloaliphatisch gebundenen und insbesondere die von Hexamethylendiisocyanat oder Isophorondiisocyanat abgeleiteten. Bei Uretdiondiisocyanaten handelt es sich um cyclische Dirnerisierungsprodukte von Diisocyanaten.
   Die Uretdiondiisocyanate können in den erfindungsgemäßen Zubereitungen als alleinige Komponente oder im Gemisch mit anderen Polyisocyanaten, insbesondere den unter 1) genannten, eingesetzt werden.
3) Biuretgruppen aufweisende Polyisocyanate mit aromatisch, cycloaliphatisch oder aliphatisch gebundenen, bevorzugt cycloaliphatisch oder aliphatisch gebundenen Isocyanatgruppen, insbesondere Tris(6-isocyanatohexyl)biuret oder dessen Gemische mit seinen höheren Homologen. Diese Biuretgruppen aufweisenden Polyisocyanate weisen im allgemeinen einen NCO-Gehalt von 18 bis 22 Gew.-% und eine mittlere NCO-Funktionalität von 2,8 bis 4,5 auf.
4) Urethan- und/oder Allophanatgruppen aufweisende Polyisocyanate mit aromatisch, aliphatisch oder cycloaliphatisch gebundenen, bevorzugt aliphatisch oder cycloaliphatisch gebundenen Isocyanatgruppen, wie sie beispielsweise durch Umsetzung von überschüssigen Mengen an Hexamethylendiisocyanat oder an Isophorondiisocyanat mit ein- oder mehrwertigen Alkoholen wie z.B. Methanol, Ethanol, *iso*-Propanol, n-Propanol, n-Butanol, *iso*-Butanol, *sek*-Butanol, *tert-*Butanol, n-Hexanol, n-Heptanol, n-Octanol, n-Decanol, n-Dodecanol (Laurylalkohol), 2-Ethylhexanol, n-Pentanol, Stearylalkohol, Cetylalkohol, Laurylalkohol, Ethylenglykolmonomethylether, Ethylenglykolmonoethylether, 1,3-Propandiolmonomethylether, Cyclopentanol, Cyclohexanol, Cyclooctanol, Cyclododecanol oder mehrwertige Alkohole, wie sie oben bei den Polyesterolen aufgeführt sind, oder deren Gemischen erhalten werden können. Diese Urethan- und/oder Allophanatgruppen aufweisenden Polyisocyanate haben im allgemeinen einen NCO-Gehalt von 12 bis 20 Gew.-% und eine mittlere NCO-Funktionalität von 2,5 bis 4,5.
5) Oxadiazintriongruppen enthaltende Polyisocyanate, vorzugsweise von Hexamethylendüsocyanat oder Isophorondiisocyanat abgeleitet. Solche Oxadiazintriongruppen enthaltenden Polyisocyanate sind aus Diisocyanat und Kohlendioxid herstellbar.
6) Iminooxadiazindiongruppen enthaltende Polyisocyanate, vorzugsweise von Hexamethylendüsocyanat oder Isophorondiisocyanat abgeleitet. Solche Iminooxadiazindiongrupperi enthaltenden Polyisocyanate sind aus Diisocyanaten mittels spezieller Katalysatoren herstellbar.
7) Uretonimin-modifizierte Polyisocyanate.

Die Polyisocyanate 1) bis 7) können im Gemisch, gegebenenfalls auch im Gemisch mit Diisocyanaten, eingesetzt werden.

Die Isocyanatgruppen können auch in verkappter Form vorliegen. Als Verkappungsmittel für NCO-Gruppen eignen sich z.B. Oxime, Phenole, Imidazole, Pyrazole, Pyrazolinone, Diketopiperazine, Caprolactam, Malonsäureester oder Verbindungen, wie sie genannt sind in den Veröffentlichungen von Z.W. Wicks, Prog. Org. Coat. 3 (1975) 73-99 und Prog. Org. Coat 9 (1981), 3 - 28 sowie in Houben-Weyl, Methoden der Organischen Chemie, Bd. XIV/2, 61 ff. Georg Thieme Verlag, Stuttgart 1963.

Unter Verkappungs- bzw. Blockierungsmitteln werden dabei Verbindungen verstanden, die isocyanatgruppen in blockierte (verkappte bzw. geschützte) isocyanatgruppen umwandeln, die dann unterhalb der sogenannten Deblockierungstemperatur nicht die üblichen Reaktionen einer freien Isocyanatgruppe zeigen. Solche Verbindungen mit blokkierten Isocyanatgruppen kommen üblicherweise in Dual-Cure-Beschichtungsmitteln zur Anwendung, die über Isocyanatgruppenhärtung endgehärtet werden.

Epoxidverbindungen sind solche mit mindestens einer, bevorzugt mit mindestens zwei, vorzugsweise zwei oder drei Epoxidgruppen im Molekül.

In Betracht kommen z.B. epoxidierte Olefine, Glycidylester (z.B. Glycidyl(meth)acrylat) von gesättigten oder ungesättigten Carbonsäuren oder Glycidylether aliphatischer oder aromatische Polyole. Derartige Produkte werden im Handel in großer Zahl angeboten. Besonders bevorzugt sind Polyglycidylverbindungen vom Bisphenol A-, -F- oder -B-Typ und Glycidylether mehrfunktioneller Alkohole, z.B. des Butandiol, des 1,6-Hexandiol, des Glycerin und des Pentaerythrit. Beispiele für derartige Polyepoxidverbindungen sind Epikote^{®} 812 (Epoxidwert: ca. 0,67 mol/100g) und Epikote^{®} 828 (Epoxidwert: ca. 0,53 mol/100g), Epikote^{®} 1001, Epikote^{®} 1007 und Epikote^{®} 162 (Epoxidwert: ca. 0,61 mol/100g) der Firma Resolution, Rütapox® 0162 (Epoxidwert: ca. 0,58 mol/100g), Rütapox^{®} 0164 (Epoxidwert: ca. 0,53 mol/100g) und Rütapox^{®} 0165 (Epoxidwert: ca. 0,48 mol/100g) der Firma Bakelite AG, Araldit^{®} DY 0397 (Epoxidwert: ca. 0,83 mol/100g) der Firma Vantico AG.

Carbonatverbindungen sind solche mit mindestens einer, bevorzugt mit mindestens zwei, vorzugsweise zwei oder drei Carbonatgruppen im Molekül, die bevorzugt endständige C₁-C₂₀-Alkylcarbonatgruppen enthalten, besonders bevorzugt endständiges C₁-C₄-Alkylcarbonatgruppen, ganz besonders bevorzugt endständiges Methylcarbonat, Ethylcarbonat oder n-Butylcarbonat.

Als Komponenten (B) kommen weiterhin Verbindungen in Frage, mit aktiven Methylol- oder Alkylalkoxygruppen, insbesondere Methylalkoxygruppen, an Aminoplastvernetzem, wie z.B veretherte Umsetzungsprodukte von Formaldehyd mit Aminen, wie Melamin, Harnstoff etc., Phenol/Formaldehydaddukte, Siloxan oder Silangruppen und Anhydride ein, wie sie z.B. in US 5,770,650 beschrieben sind, ein.

Unter den technisch weit verbreiteten und bekannten, bevorzugten Aminoplasten sind besonders bevorzugt Harnstoffharze und Melaminharze, wie z.B. Hamstoff-Formaldehyd-Harze, Melamin-Formaldehyd-Harze, Melamin-Phenol-Formaldehyd-Harze oder Melamin-Harnstoff-Formaldehyd-Harze, verwendbar.

Als Harnstoffharze sind solche geeignet, die durch Umsetzung von Harnstoffen mit Aldehyden erhältlich sind und gegebenenfalls modifiziert werden können.

Als Harnstoffe sind Harnstoff, N-substituierte oder N,N'-disubstituierte Harnstoffe geeignet, wie z.B. N-Methylharnstoff, N-Phenylhamstoff, N,N'-Dimethylhamstoff, Hexamethylendiharnstoff, N,N'-Diphenylharnstoff, 1,2-Ethylendiharnstoff, 1,3-Propylen-diharnstoff, Diethylentriharnstoff, Dipropylentrihamstoff, 2-Hydroxypropylendiharnstoff, 2-lmidazolidinon (Ethylenharnstoff), 2-Oxohexahydropyrimidin (Propylenharnstoff) oder 2-Oxo-5-Hydroxyhexahydropyrimidin (5-Hydroxypropylenharnstoff).

Harnstoffharze können gegebenenfalls teilweise oder vollständig modifiziert werden, z.B. durch Umsetzung mit mono- od. polyfunktionellen Alkoholen, Ammoniak bzw. Aminen (kationisch modifizierte Harnstoffharze) oder mit (Hydrogen)sulfiten (anionisch modifizierte Hamstoffharze), insbesondere geeignet sind die alkoholmodifizierten Harnstoffharze.

Als Alkohole kommen für die Modifizierung C₁ - C₆-Alkohole in Frage, bevorzugt C₁ - C₄-Alkohol und insbesondere Methanol, Ethanol, *iso*-Propanol, n-Propanol, n-Butanol, *iso*-Butanol und *sek*-Butanol.

Als Melaminharze sind solche geeignet, die durch Umsetzung von Melamin mit Aldehyden erhältlich sind und gegebenenfalls teilweise oder vollständig modifiziert werden können.

Als Aldehyde sind insbesondere Formaldehyd, Acetaldehyd, *Iso*-Butyraldehyd und Glyoxal geeignet.

Melamin-Formaldehyd-Harze sind Reaktionsprodukte der Umsetzung von Melamin mit Aldehyden, z.B. den o.g. Aldehyden, insbesondere Formaldehyd. Gegebenenfalls werden die erhaltenen Methylol-Gruppen durch Veretherung mit den oben genannten ein- oder mehrwertigen Alkoholen modifiziert. Weiterhin können die Melamin-Formaldehyd-Harze auch wie oben beschrieben durch Reaktion mit Aminen, Aminocarbonsäuren oder Sulfiten modifiziert werden.

Durch Einwirkung von Formaldehyd auf Mischungen von Melamin und Harnstoff beziehungsweise auf Mischungen aus Melamin und Phenol entstehen erfindungsgemäß ebenfalls verwendbare Melamin-Harnstoff-Fonilaldehyd-Harze beziehungsweise Melamin-Phenol-Formaldehyd-Harze.

Die Herstellung der genannten Aminoplaste erfolgt nach an sich bekannten Verfahren.

Besonders genannte Beispiele sind Melamin-Formaldehyd-Harze, einschließlich monomerer oder polymerer Melaminharze und teilweise oder vollständig alkylierte Melaminharze, Harnstoff-Harze, z.B. Methylolharnstoffe wie Formaldehyd-Harnstoff-Harze, Alkoxyharnstoffe wie butylierte Formaldehyd-Harnstoff-Harze, aber auch N-Methylolacrylamid-emulsionen, *iso*-Butoxy methyl acrylamid-emulsionen, Polyanhydride, wie z.B. Polybemsteinsäureanhydrid, und Siloxane oder Silane, z.B. Dimethyldimethoxysilane.

Besonders bevorzugt sind Aminoplastharze wie Melamin-Formaldehyd-Harze oder Formaldehyd-Hamstoff-Harze.

Ebenfalls offenbart wird ein Verfahren zur Beschichtung von Substraten, in dem man eine erfindungsgemäße Beschichtungsmasse einsetzt.

Die Beschichtung der Substrate erfolgt nach üblichen, dem Fachmann bekannten Verfahren, wobei man wenigstens eine erfindungsgemäße Beschichtungsmasse oder diese enthaltende Lackformulierung auf das zu beschichtende Substrat in der gewünschten Stärke aufbringt und die flüchtigen Bestandteile der Beschichtungsmasse, gegebenenfalls unter Erhitzen, entfernt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen oder Gießen erfolgen. Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man die erfindungsgemäßen Beschichtungsmassen oder diese enthaltende Lackformulierungen, gegebenenfalls mit weiteren lacktypischen Additiven und thermisch härtbaren Harzen versetzt, auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160 °C, thermisch behandelt.

Das Verfahren zum Beschichten von Substraten kann auch so durchgeführt werden, daß nach dem Aufbringen der erfindungsgemäßen Beschichtungsmasse oder Lackformulierungen zunächst bei Temperaturen bis zu 160°C, bevorzugt zwischen 60 und 160°C, thermisch behandelt und anschließend mit Elektronenstrahlen oder UV Belichtung unter Sauerstoff oder bevorzugt unter Inertgas gehärtet wird.

Die Härtung der auf dem Substrat gebildeten Filme kann gewünschtenfalls ausschließlich thermisch erfolgen. Im allgemeinen und bevorzugt härtet man die Beschichtungen jedoch sowohl durch Bestrahlung mit energiereicher Strahlung als auch thermisch.

Gegebenenfalls kann, wenn mehrere Schichten des Beschichtungsmittels übereinander aufgetragen werden, nach jedem Beschichtungsvorgang eine thermische und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier.

Diese können auch in jeweils unterschiedlichen Wellenlängebereichen strahlen.

Die Härtung kann auch zusätzlich zur oder anstelle der thermischen Härtung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.

Die Bestrahlung kann gegebenenfalls auch unter Ausschluß von Sauerstoff, z.B. unter Inertgas-Atmosphäre, durchgeführt werden. Als Inertgase eignen sich vorzugsweise Stickstoff, Edelgase, Kohlendioxid oder Verbrennungsgase. Desweiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.
Ein weiterer Gegenstand der Erfindung ist ein Verfahren zur Beschichtung von Substraten, wobei man
i) ein Substrat mit einer Beschichtungsmasse, wie zuvor beschrieben, beschichtet,
ii) flüchtige Bestandteile der Beschichtungsmasse zur Filmbildung unter Bedingungen entfernt, bei denen der Initiator (P) im wesentlichen noch keine freien Radikale ausbildet,
iii) gegebenenfalls den in Schritt ii) gebildeten Film mit energiereicher Strahlung bestrahlt, wobei der Film vorgehärtet wird, und anschließend gegebenenfalls den mit dem vorgehärteten Film beschichteten Gegenstand mechanisch bearbeitet oder die Oberfläche des vorgehärteten Films mit einem anderen Substrat in Kontakt bringt,
iv) dem Film thermisch endhärtet.

Dabei können die Schritte iv) und iii) auch in umgekehrter Reihenfolge durchgeführt, d. h. der Film kann zuerst thermisch und dann mit energiereicher Strahlung gehärtet werden.

Die erfindungsgemäßen Beschichtungsmassen und Lackformulierungen eignen sich besonders zum Beschichten von Substraten wie Holz, Papier, Textil, Leder, Vlies, Kunststoffoberflächen, Glas, Keramik, mineralischen Baustoffen, wie Zement-Formsteine und Faserzementplatten, oder Metallen oder beschichteten Metallen, bevorzugt von Kunststoffen oder Metallen, die beispielsweise auch als Folien vorliegen können.

Besonders bevorzugt eignen sich die erfindungsgemäßen Beschichtungsmassen als oder in Außenbeschichtungen, also solche Anwendungen, die dem Tageslicht ausgesetzt sind, bevorzugt von Gebäuden oder Gebäudeteilen, Innenbeschichtungen, Straßenmarkierungen, Beschichtungen auf Fahrzeugen und Flugzeugen. Insbesondere werden die erfindungsgemäßen Beschichtungsmassen als oder in Automobilklar- und -decklacke(n) eingesetzt.

Ein weiterer erfindungsgemäßer Gegenstand ist die Verwendung von α-(1'-Hydroxyalkyl)acrylaten in Beschichtungsmassen für Dual-Cure Anwendungen.

Ein weiterer erfindungsgemäßer Gegenstand ist die Verwendung von Verbindungen der Formel (V) oder (VII) in der Strahlungshärtung.

Die folgenden Beispiele sollen die Eigenschaften der Erfindung erläutern, ohne sie aber einzuschränken.

### Beispiele

Als "Teile" oder "%" seien in dieser Schrift, wenn nicht anders angegeben, "Gewichtsteile" oder "Gewicht%" verstanden.

Die Erichsentiefung wurde nach DIN 53156 bestimmt und ist ein Maß für die Flexibilität und Elastizität. Die Angabe erfolgt in Millimeter (mm). Hohe Werte bedeuten hohe Flexibilität. Die Filme zur Bestimmung der Erichsentiefung wurden wenn nicht anders angegeben mit Hilfe einer Spiralraken auf Blech aufgetragen. Die Schichtdicke nach der Belichtung betrug wenn nicht anders angegeben 40 µm.

Die Pendelhärte wurde nach DIN 53157 bestimmt und ist ein Maß für die Härte der Beschichtung. Die Angabe erfolgt in Sekunden (s). Hohe Werte bedeuten dabei hohe Härte. Die Filme zur Bestimmung der Pendelhärte wurden wenn nicht anders angegeben mit Hilfe einer Kastenrakel auf Glas aufgetragen. Die Schichtdicke nach der Belichtung-betrug wenn nicht anders angegeben 70 µm.

Die OH-Zahl wurde gem. DIN 53240 bestimmt.

### Beispiel 1:

148 Teile Trimethylolpropan triacrylat, 17 Teile Diaza-bicyclo-octan, 100 Teile Tetrahydrofuran und 150 Teile Formaldehydlösung (36%ig) wurden in einem Reaktionskolben zusammengegeben und 2 Stunden bei 60°C gerührt.
Dann gab man 100 Teile Diethylether zu, schüttelte 2 mal mit 100 Teilen 1 N Salzsäure und 1 mal mit wässriger Natriumchloridlösung aus, trennte die Phasen, trocknete die organische Phase über Natriumsulfat, setzte 1 Teil Phenothiazin zu und destillierte das Lösungsmittel ab.

Es resultierte eine klare, schwach gelbliche Flüssigkeit, die im IR Spektrum eine deutliche OH Bande bei 3500 cm-1 (OH Zahl 109 mgKOH/g) und eine Verschiebung der unsubstituierten Acrylatbande bei 810 cm⁻¹ nach 820 cm⁻¹ aufwies. Im GC Chromatogramm war kein freies Trimethylolpropan triacrylat mehr vorhanden.

### Beispiel 2:

148 Teile Trimethylolpropan triacrylat, 5,6 Teile Diaza-bicyclo-octan, 100 Teile Tetrahydrofuran und 50 Teile Formaldehydlösung (36%ig) wurden in einem Reaktionskolben zusammengegeben und 2 Stunden bei 60°C gerührt.
Dann gab man 100 Teile Diethylether zu, schüttelte 2 mal mit 100 Teilen 1 N Salzsäure und 1 mal mit wässriger Natriumchloridlösung aus, trennte die Phasen, trocknete die organische Phase über Natriumsulfat, setzte 1 Teil Phenothiazin zu und destillierte das Lösungsmittel ab.

Es resultierte eine klare Flüssigkeit mit einer Viskosität von 510 mPas, die im IR Spektrum eine deutliche OH Bande bei 3500 cm⁻¹ aufwies (OH Zahl 63 mgKOH/g). Im GC Chromatogramm waren noch 23 Flächenprozent freies Trimethylolpropan triacrylat vorhanden.

### Beispiel 3:

302 Teile Triacrylat von siebenfach ethoxyliertem Trimethylolpropan, 17 Teile Diaza-bicyclo-octan, 100 Teile Tetrahydrofuran und 150 Teile Formaldehydlösung (36%ig) wurden in einem Reaktionskolben zusammengegeben und 2 Stunden bei 60°C gerührt.

Dann gab man 100 Teile Diethylether zu, schüttelte 2 mal mit 100 Teilen 1 N Salzsäure und 1 mal mit wässriger Natriumchloridlösung aus, trennte die Phasen, trocknete die organische Phase über Natriumsulfat, setzte 1 Teil Phenothiazin zu und destillierte das Lösungsmittel ab.

Es resultierte eine klare, schwach gelbliche Flüssigkeit, die im IR Spektrum eine deutliche OH Bande bei 3500 cm⁻¹ aufwies.

### Beispiel 4:

500 Teile eines kommerziell erhältlichen Polyesteracrylats (Laromer^{®} PE 55F, BASF Aktiengesellschaft), 14 Teile Diaza-bicyclo-octan, 100 Teile Tetrahydrofuran und 125 Teile Formaldehydlösung (36%ig) werden in einem Reaktionskolben zusammengegeben und 2 Stunden bei 60°C gerührt.
Dann gibt man 100 Teile Diethylether zu, schüttelt 2 mal mit 100 Teilen 1 N Salzsäure und 1 mal mit wässriger Natriumchloridlösung aus, trennt die Phasen, trocknet die organische Phase über Natriumsulfat, setzt 1 Teil Phenothiazin zu und destilliert das Lösungsmittel ab.
Es resultiert eine klare, schwach gelbliche Flüssigkeit, die im IR Spektrum eine deutliche OH Bande bei 3500 cm⁻¹ aufweist.

### Beispiel 5:

215 Teile eines kommerziell erhältlichen Epoxyacrylats ((Laromer^{®} EA 81, BASF Aktiengesellschaft), 11 Teile Diaza-bicyclo-ocfan, 100 Teile Tetrahydrofuran und 100 Teile Formaldehydlösung (36%ig) werden in einem Reaktionskolben zusammengegeben und 2 Stunden bei 60°C gerührt.

Dann gibt man 100 Teile Diethylether zu, schüttelt 2 mal mit 100 Teilen 1 N Salzsäure und 1mal mit wässriger Natriumchloridlösung aus, trennt die Phasen, trocknet die organische Phase über Natriumsulfat, setzt 1 Teil Phenothiazin zu und destilliert das Lösungsmittel ab.

Es resultiert eine klare, schwach gelbliche Flüssigkeit, die im IR Spektrum gegenüber der Ausgangsverbindung eine deutliche erhöhte OH Bande bei 3500 cm⁻¹ aufweist.

### Beispiel 6:

750 Teile eines kommerziell erhältlichen Urethanacrylats ((Laromer^{®} UA19T, BASF Aktiengesellschaft), 11 Teile Diaza-bicyclo-octan, 100 Teile Tetrahydrofuran und 100 Teile Formaldehydlösung (36%ig) werden in einem Reaktionskolben zusammengegeben und 2 Stunden bei 60°C gerührt.

Dann gibt man 100 Teile Diethylether zu, schüttelt 2 mal mit 100 Teilen 1 N Salzsäure und 1 mal mit wässriger Natriumchloridlösung aus, trennt die Phasen, trocknet die organische Phase über Natriumsulfat, setzt 1 Teil Phenothiazin zu und destilliert das Lösungsmittel ab.

Es resultiert eine klare, schwach gelbliche Flüssigkeit, die im IR Spektrum neben der NH-Bande eine deutliche OH Bande bei 3500 cm⁻¹ aufweist.

### Beispiel 7: Dual Cure Anwendung

10 Teile des nach Beispiel 1 hergestellten α-Hydroxyalkyl-acrylats wurden mit 3,8 Teilen eines Trimeren Isocyanurats von Hexamethylendiisocyanat gemischt und in einer Schichtdicke von ca. 5 µm auf ein KBr IR Plättchen aufgetragen. Das IR Spektrum zeigte die OH Banden bei 3500 cm⁻¹, die NCO- Banden bei 2250 cm⁻¹ und die Acrylatbanden bei 820 cm⁻¹. Bei Temperung des IR Kristalls bei 180°C für 2 Stunden, verschwanden die NCO Bande bei 2250 cm⁻¹ fast vollständig, die Acrylatbande reduzierte sich und die OH Bande hatte sich nach 3350 cm⁻¹ (NH) verschoben. Durch anschließende Belichtung verschwand die Acrylatbande ebenfalls nahezu vollständig. Ähnliche Resultate erhielt man auch, wenn man zuerst belichtet und dann bei 180°C erhitzt.

### Beispiel 8:

10 Teile des nach Beispiel 1 hergestellten α-Hydroxyalkyl-acrylats wurden mit 0,4 Teilen des Photoinitiators Darocure^{®} 1173 (CIBA Specialities) versetzt und in einer Schichtdicke von ca. 50 µm auf eine Glasplatte aufgetragen und mit 1200 mJ/cm² belichtet. Es resultierte eine Pendelhärte von 140 s und eine Erichsentiefung von 0,5 mm.

### Vergleichsbeispiel 1:

Mit den gleichen Bedingungen wie in Beispiel 8 wurde Trimethylolpropan triacrylat anstatt des Umsetzungsprodukts aus Beispiel 1 prozessiert. Es resultierte eine Pendelhärte von 172 s und eine Erichsentiefung von 0.

Beispiel 8 und Vergleichsbeispiel 1 zeigen, dass man mit den erfindungsgemäßen alpha-Hydroxyacrylaten ähnliche Eigenschaften nach UV Belichtung bezüglich Härte und Flexibilität erhält wie mit unsubstituiertem Acrylat und Beispiel 7 zeigt, dass man den erfindungsgemäßen Produkten Dual Care Lacke herstellen kann.

## Patentansprüche

1. Verbindungen der Formel (V), 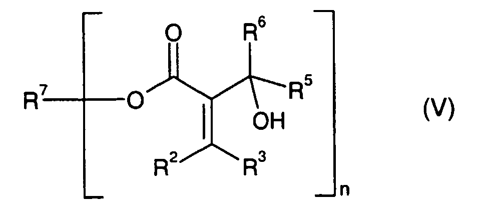 worin
R² und R³ unabhängig voneinander C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können,
R² und/oder R³ zusätzlich Wasserstoff, gegebenenfalls durch Aryl, Alkyl, Aryloxy, Alkyloxy, Heteroatome und/oder Heterocyclen substituiertes C₁ - C₁₈-Alkoxy oder -COOR⁴,
R² kann zusätzlich zusammen mit R¹ einen Ring bilden, in diesem Fall kann R² eine Carbonylgruppe bedeuten, so daß die Gruppe COOR¹ und R² gemeinsam eine Säureanhydridgruppe -(CO)-O-(CO)- bilden,
R⁴ C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können,
R⁵ und R⁶ unabhängig voneinander Wasserstoff, C₁- C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, oder können gemeinsam einen Ring bilden,
n eine positive ganze Zahl von 3 bis 10,
R⁷ einen n-wertigen organischen Rest mit 1 bis 50 Kohlenstoffatomen, der unsubstituiert oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxy oder hydroxysubstituiertem C₁-C₈-Alkyl substituiert sein und/oder eine oder mehrere -(CO)-, -O(CO)O-, -(NH)(CO)O-, -O(CO)(NH)-, -O(CO)- oder -(CO)O-Gruppen aufweisen kann.

2. Verbindungen gemäß Anspruch 1, **dadurch gekennzeichnet, daß**
n 3 oder 4 ist und
R⁷ abgeleitet ist von einem n-wertigen Alkohol durch Entfernung von n Hydroxygruppen,
wobei es sich bei dem n-wertigen Alkohol um Trimethylolpropan, Pentaerythrit oder ein bis zwanzigfach ethoxyliertes Trimethylolpropan handelt.

3. Beschichtungsmassen, enthaltend
- mindestens eine Verbindung der Formel (V), wie in Anspruch 1 definiert, oder der Formel (VII), wie in Anspruch 10 definiert, und
- mindestens einen Photoinitiator (P).

4. Beschichtungsmassen nach Anspruch 3, enthaltend zusätzlich
- mindestens einen Reaktivverdünner und/oder
- mindestens eine multifunktionelle, polymerisationsfähige Verbindung.

5. Beschichtungsmassen nach Anspruch 3 oder 4, enthaltend zusätzlich
- mindestens eine Verbindung (B) mit mindestens einer gegenüber Hydroxy (-OH) reaktiven Gruppe.

6. Verfahren zur Beschichtung von Substraten, **dadurch gekennzeichnet, daß** man eine Beschichtungsmasse gemäß einem der Ansprüche 3 bis 5 einsetzt.

7. Substrat, beschichtet mit einer Beschichtungsmasse gemäß einem der Ansprüche 3 bis 5.

8. Verfahren zur Herstellung von Verbindungen der Formel (V) 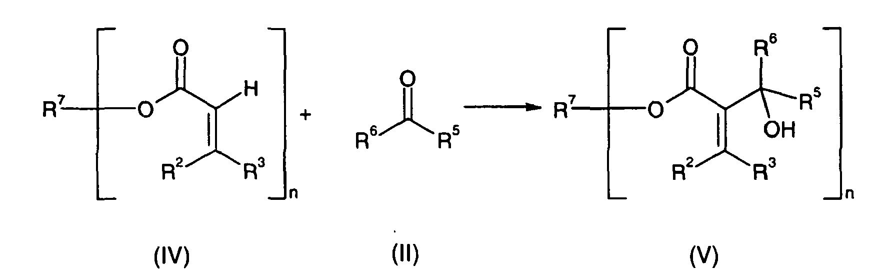 wie in Anspruch 1 definiert, wobei n zusätzlich 2 sein kann, **dadurch gekennzeichnet, daß** die Verbindung (II) ein Aldehyd R⁵-CHO ist und man diesen Aldehyd in freier Form einsetzt, so daß in Formalen der Formel (R⁵-CHO)_{w}, worin w eine positive ganze Zahl ist, w ≤ 20 ist.

9. Verwendung von α-(1'-Hydroxyalkyl)acrylaten in Beschichtungsmassen für Dual-Cure Anwendungen.

10. Verwendung von Verbindungen der Formel (V) wie in Anspruch 8 definiert oder (VII) 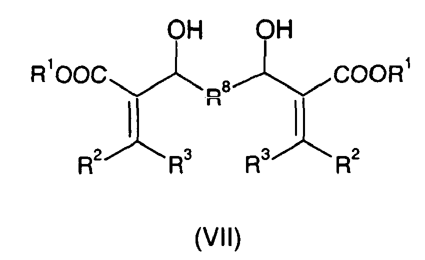 worin R² und R³ definiert sind wie in Anspruch 1,
R¹ C₁- C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein kann und
R⁸ unsubstituiertes oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₆-C₁₂-Arylen, C₃-C₁₂-Cycloalkylen, Ci-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, -O(CO)(NH)-, -O(CO)- oder -(CO)O-Gruppen unterbrochenes C₂-C₂₀-Alkylen oder eine Einfachbindung bedeuten,
in der Strahlungshärtung.

## Claims

1. A compound of the formula (V), 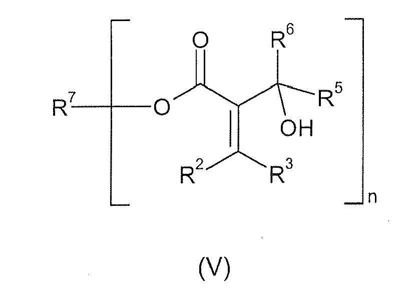 in which
R² and R³ independently of one another are C₁-C₁₈ alkyl, C₂-C₁₈ alkyl if appropriate interrupted by one or more oxygen and/or sulfur atoms and/or one or more substituted or unsubstituted imino groups, C₂-C₁₈ alkenyl, C₆-C₁₂ aryl, C₅-C₁₂ cycloalkyl or a five- to six-membered oxygen-, nitrogen- and/or sulfur-containing heterocycle, it being possible for each of the stated radicals to be substituted by aryl, alkyl, aryloxy, alkyloxy, heteroatoms and/or heterocycles,
R² and/or R³ are/is additionally hydrogen, C₁-C₁₈ alkoxy optionally substituted by aryl, alkyl, aryloxy, alkyloxy, heteroatoms and/or heterocycles, or -COOR⁴,
R² may additionally together with R¹ form a ring, in which case R² can be a carbonyl group, so that the group COOR¹ and R² together form an acid anhydride group -(CO)-O-(CO)-,
R⁴ is C₁-C₁₈ alkyl, C₂-C₁₈ alkyl if appropriate interrupted by one or more oxygen and/or sulfur atoms and/or one or more substituted or unsubstituted imino groups, C₂-C₁₈ alkenyl, C₆-C₁₂ aryl, C₅-C₁₂ cycloalkyl or a five- to six-membered oxygen-, nitrogen- and/or sulfur-containing heterocycle, it being possible for each of the stated radicals to be substituted by aryl, alkyl, aryloxy, alkyloxy, heteroatoms and/or heterocycles,
R⁵ and R⁶ independently of one another are hydrogen, C₁-C₁₈ alkyl, C₂-C₁₈ alkyl if appropriate interrupted by one or more oxygen and/or sulfur atoms and/or one or more substituted or unsubstituted imino groups, C₂-C₁₈ alkenyl, C₆-C₁₂ aryl, C₅-C₁₂ cycloalkyl or a five- to six-membered oxygen-, nitrogen- and/or sulfur-containing heterocycle, it being possible for each of the stated radicals to be substituted by aryl, alkyl, aryloxy, alkyloxy, heteroatoms and/or heterocycles, or may together form a ring,
n is a positive integer from 3 to 10, and
R⁷ is an n-valent organic radical having 1 to 50 carbon atoms which can be unsubstituted or substituted by halogen, C₁-C₈ alkyl, C₂-C₈ alkenyl, carboxyl, carboxy-C₁-C₈ alkyl, C₁-C₂₀ acyl, C₁-C₈ alkoxy, C₆-C₁₂ aryl, hydroxyl or hydroxy-substituted C₁-C₈ alkyl and/or can contain one or more -(CO)-, -O(CO)O-, -(NH)(CO)O-, -O(CO)(NH)-, -O(CO)- or -(CO)O- groups.

2. The compound according to claim 1, wherein n is 3 or 4 and
R⁷ is derived from an n-hydric alcohol by removing n hydroxyl groups,
the n-hydric alcohol being trimethylolpropane, pentaerythritol or a singly to vigintuply ethoxylated trimethylolpropane.

3. A coating composition comprising
- at least one compound of the formula (V) as defined in claim 1, or of the formula (VII) as defined in claim 10, and
- at least one photoinitiator (P).

4. The coating composition according to claim 3, further comprising
- at least one reactive diluent and/or
- at least one polyfunctional polymerizable compound.

5. The coating composition according to claim 3 or 4, further comprising
- at least one compound (B) containing at least one hydroxy (-OH)-reactive group.

6. A method of coating substrates, wherein a coating composition according to any one of claims 3 to 5 is used.

7. A substrate coated with a coating composition according to any one of claims 3 to 5.

8. A process for preparing a compound of the formula (V) 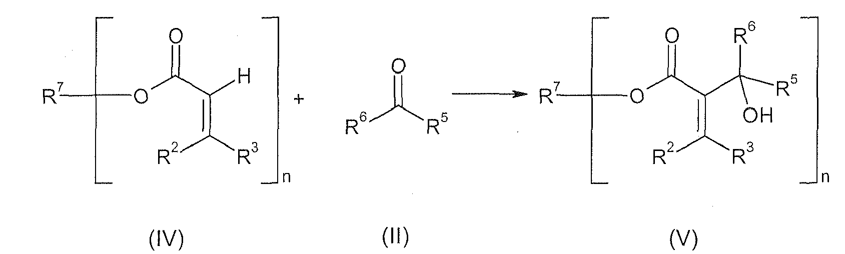 as defined in claim 1, it being possible for n to be additionally 2, wherein the compound (II) is an aldehyde R⁵-CHO and is used in free form so that in formals of the formula (R⁵-CHO)_{w}, in which w is a positive integer, w is ≤ 20.

9. The use of α-(1'-hydroxyalkyl)acrylates in coating compositions for dual-cure applications.

10. The use of compounds of the formula (V) as defined in claim 8 or (VII) 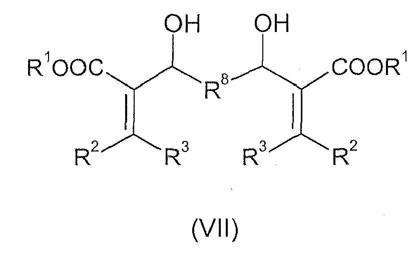 in which R² and R³ are as defined in claim 1,
R¹ is C₁-C₁₈ alkyl, C₂-C₁₈ alkyl if appropriate interrupted by one or more oxygen and/or sulfur atoms and/or one or more substituted or unsubstituted imino groups, C₂-C₁₈ alkenyl, C₆-C₁₂ aryl, C₅-C₁₂ cycloalkyl or a five- to six-membered oxygen-, nitrogen- and/or sulfur-containing heterocycle, it being possible for each of the stated radicals to be substituted by aryl, alkyl, aryloxy, alkyloxy, heteroatoms and/or heterocycles, and
R⁸ is unsubstituted or halogen-, C₁-C₈ alkyl-, C₂-C₈ alkenyl-, carboxyl-, carboxy-C₁-C₈ alkyl-, C₁-C₂₀ acyl-, C₁-C₈ alkoxy-, C₆-C₁₂ aryl-, hydroxyl- or hydroxy-substituted C₁-C₈ alkyl-substituted C₆-C₁₂ arylene, C₃-C₁₂ cycloalkylene or C₁-C₂₀ alkylene or is C₂-C₂₀ alkylene interrupted by one or more oxygen and/or sulfur atoms and/or one or more substituted or unsubstituted imino groups and/or by one or more -(CO)-, -O(CO)O-, -(NH)(CO)O-, -O(CO)(NH)-, -O(CO)- or -(CO)O-groups or is a single bond
in radiation curing.

## Revendications

1. Composés de formule (V) : 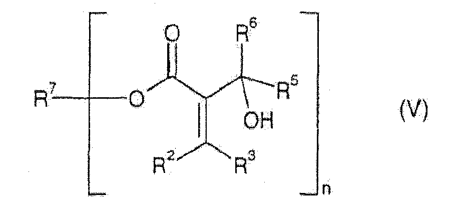 dans laquelle :
R² et R³ représentent, indépendamment l'un de l'autre, un alkyle en C₁-C₁₈, ou un groupement alkyle en C₂-C₁₈, alcényle en C₂-C₁₈, aryle en C₆-C₁₂, cycloalkyle en C₅-C₁₂ éventuellement interrompu par un ou plusieurs atomes d'oxygène et/ou de soufre, et/ou par un ou plusieurs groupements imino substitués ou non substitués, ou représentent un hétérocycle à cinq à six éléments présentant des atomes d'oxygène, d'azote et/ou de soufre, les radicaux cités pouvant être substitués, respectivement, par un groupement aryle, alkyle, aryloxy, alkyloxy, des hétéroatomes et/ou des hétérocycles,
R² et/ou R³ représentent, en outre, l'hydrogène, un alcoxy en C₁-C₁₈ éventuellement substitué par un groupement aryle, alkyle, aryloxy, alkyloxy, des hétéroatomes et/ou des hétérocycles, ou représentent un groupement -COOR⁴,
R² peut encore, conjointement avec R¹, former un cycle, R² pouvant dans ce cas représenter un groupement carbonyle, de sorte que les groupements COOR¹ et R² forment conjointement un groupement anhydride d'acide de type -(CO)-O-(CO)-,
R⁴ représente un alkyle en C₁-C₁₈, ou un groupement alkyle en C₂-C₁₈, alcényle en C₂-C₁₈, aryle en C₆-C₁₂, cycloalkyle en C₅-C₁₂ éventuellement interrompu par un ou plusieurs atomes d'oxygène et/ou de soufre et/ou par un ou plusieurs groupements imino substitués ou non substitués, ou représente un hétérocycle à cinq ou six éléments présentant des atomes d'oxygène, d'azote et/ou de soufre, les radicaux cités pouvant être substitués, respectivement, par un groupement aryle, alkyle, aryloxy, alkyloxy, des hétéroatomes et/ou des hétérocycles,
R⁵ et R⁶ représentent, indépendamment l'un de l'autre, l'hydrogène, un alkyle en C₁-C₁₈, ou un groupement alkyle en C₂-C₁₈, alcényle en C₂-C₁₈, aryle en C₆-C₁₂, cycloalkyle en C₅-C₁₂ éventuellement interrompu par un ou plusieurs atomes d'oxygène et/ou de soufre et/ou par un ou plusieurs groupements imino substitués ou non substitués, ou représentent un hétérocycle à cinq ou six éléments présentant des atomes d'oxygène, d'azote et/ou de soufre, les radicaux cités pouvant être substitués, respectivement, par un groupement aryle, alkyle, aryloxy, alkyloxy, des hétéroatomes et/ou des hétérocycles, ou peuvent former conjointement un cycle,
n est un nombre entier positif de 3 à 10,
R⁷ représente un radical organique de valence n ayant 1 à 50 atomes de carbone, qui peut être non substitué ou substitué par un halogène, un groupement alkyle en C₁-C₈, alcényle en C₂-C₈, carboxy, carboxyalkyle en C₁-C₈, acyle en C₁-C₂₀, alcoxy en C₁-C₈, aryle en C₆-C₁₂, hydroxyle ou alkyle en C₁-C₈ substitué par un hydroxyle, et/ou présenter un ou plusieurs groupements -(CO)-, -O(CO)O-, -(NH)(CO)O-, -O(CO)(NH)-, -O(CO)- ou -(CO)O-.

2. Composés selon la revendication 1, **caractérisés en ce que** n est égal à 3 ou 4, et
R⁷ est dérivé d'un alcool de valence n par élimination de n groupements hydroxyle,
l'alcool de valence n en question étant le triméthylolpropane, le pentaérythritol ou un triméthylolpropane éthoxylé de une jusqu'à vingt fois.

3. Masses de revêtement, contenant :
- au moins un composé de formule (V), telle que définie dans la revendication 1, ou de formule (VII), telle que définie dans la revendication 10, et
- au moins un photoinitiateur (P).

4. Masses de revêtement selon la revendication 3, contenant en plus :
- au moins un diluant réactif, et/ou
- au moins un composé polymérisable multifonctionnel.

5. Masses de revêtement selon la revendication 3 ou 4, contenant en plus :
- au moins un composé (B) contenant au moins un groupement réactif vis-à-vis du groupement hydroxyle (-OH).

6. Procédé pour revêtir des substrats, **caractérisé en ce que** l'on utilise une masse de revêtement selon l'une quelconque des revendications 3 à 5.

7. Substrat revêtu avec une masse de revêtement selon l'une quelconque des revendications 3 à 5.

8. Procédé de fabrication de composés de formule (V) : 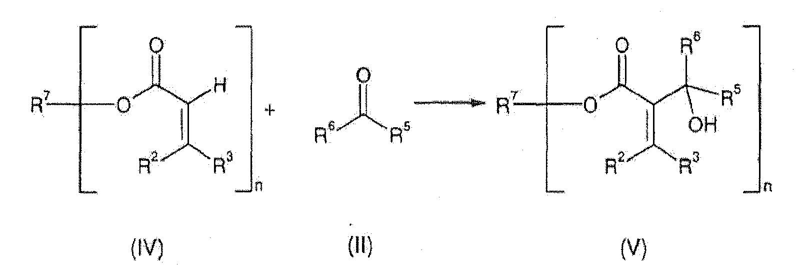 telle que définie dans la revendication 1, dans laquelle n peut en outre être égal à 2, **caractérisé en ce que** le composé (II) est un aldéhyde de type R⁵-CHO et **en ce que** l'on utilise cet aldéhyde sous forme libre, de sorte que dans les formals de formule (R⁵-CHO)_{w}, dans laquelle w est un nombre entier positif, w ait une valeur ≤ 20.

9. Utilisation de α-(1'-hydroxyalkyl)acrylates dans des masses de revêtement pour des applications à durcissement double.

10. Utilisation de composés de formule (V), telle que définie dans la revendication 8, ou de formule (VII) : 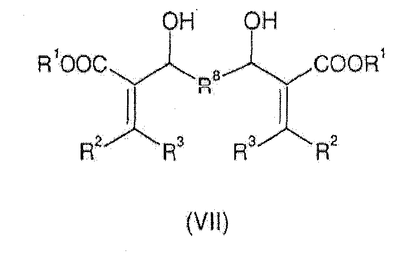 dans laquelle R² et R³ sont tels que définis dans la revendication 1,
R¹ représente un alkyle en C₁-C₁₈, ou un groupement alkyle en C₂-C₁₈, alcényle en C₂-C₁₈, aryle en C₆-C₁₂, cycloalkyle en C₅-C₁₂ éventuellement interrompu par un ou plusieurs atomes d'oxygène et/ou de soufre et/ou par un ou plusieurs groupements imino substitués ou non substitués, ou représente un hétérocycle à cinq ou six éléments présentant des atomes d'oxygène, d'azote et/ou de soufre, les radicaux cités pouvant être substitués, respectivement, par un groupement aryle, alkyle, aryloxy, alkyloxy, des hétéroatomes et/ou des hétérocycles, et
R⁸ représente un groupement arylène en C₆-C₁₂, cycloalkylène en C₃-C₁₂, alkylène en C₁-C₂₀ non substitué ou substitué par un halogène, un groupement alkyle en C₁-C₈, alcényle en C₂-C₈, carboxy, carboxyalkyle en C₁-C₈, acyle en C₁-C₂₀, alcoxy en C₁-C₈, aryle en C₆-C₁₂, hydroxyle ou par un alkyle en C₁-C₈ à substitution hydroxyle, ou représente un alkylène en C₂-C₂₀ interrompu par un ou plusieurs atomes d'oxygène et/ou de soufre et/ou par un ou plusieurs groupements imino substitués ou non substitués et/ou par un ou plusieurs groupements -(CO)-, -O(CO)O-, -(NH)(CO)O-, -O(CO)(NH)-, -O(CO)- ou - (CO)O-, ou représente une liaison simple,
à des fins de durcissement par rayonnement.
